# EUROPEAN PATENT APPLICATION

(11) **EP 3 647 425 A1**
(43) Date of publication of application: **06.05.2020**
(21) Application number: 18204167.3
(22) Date of filing: 02.11.2018
(51) Int. Cl.: C12N 15/62, C12P 7/06

(54) **RECOMBINANT MODIFIED MICROORGANISMS DISPLAYING MEMBRANE FUNCTIONALIZATION FOR VARIOUS BIOTECHNOLOGICAL APPLICATIONS**

(71) Applicant: Centre National de la Recherche Scientifique (CNRS), 75794 Paris Cedex 16 (FR); Universite d'Aix-Marseille (AMU), 13284 Marseille Cedex 07 (FR)
(72) Inventor: FIEROBE, Henri-Pierre, 13009 Marseille (FR); VITA, Nicolas, 13008 Marseille (FR); BORNE, Romain, 13009 Marseille (FR)
(74) Representative: Macquet, Christophe

(57) **Abstract**

The invention relates to a recombinant modified *Gammaproteobacteriaceae* that displays, on its cell surface, a modified Curli system comprising i) a CsgA protein or a CsgA homologous protein associated to the surface of said *Gammaproteobacteriaceae,* and ii) a scaffoldin comprising at least one cellulosomal component, said scaffolding being fused to the C-terminus of the CsgA protein. The invention also concerns uses of the recombinant modified *Gammaproteobacteriaceae* in biotechnological applications.

## Description

### TECHNICAL FIELD

The invention generally relates to the engineering, expression, secretion and cell surface exposure of heterologous cellulosomes in microorganisms. The invention aims to provide new recombinant modified microorganisms, in particular *Gammaproteobacteriaceae,* and uses thereof, for various biotechnological applications.

### BACKGROUND OF THE INVENTION

The microbial cell surface display of proteins is a widely used approach for industrial applications including vaccine development, gene therapy, cell-based diagnostics, high-throughput polypeptide library screening, whole-cell biocatalysis, bioremediation, biosensors and even biofuels production. Microbial cell surface display systems are typically based on the expression of translational fusions of open reading frames coding for a carrier protein and a desired passenger protein. According to the state of the art, systems have been developed for displaying desired proteins on the outer surface of both prokaryotic and eukaryotic cells such as Gram-positive and Gram-negative bacteria, and yeasts.

The selection of a host strain for surface display is an important factor that cannot be neglected. For Gram-negative bacteria, including *Escherichia coli,* the fragility of outer membrane caused by the display of proteins can be a problem. Gram-positive bacteria, mainly *Bacillus* and *Staphylococcus* strains, seem to be more suitable for whole-cell catalysts and whole-cell absorbents because of the rigid structure of their cell walls.

Gram-negative bacteria possess a complex cell envelope structure that comprises the cytoplasmic membrane, the periplasm and the outer membrane. This means that the surface anchoring motif, fused with the protein to be displayed, should pass through the cytoplasmic membrane and periplasm to then be addressed to the outer membrane. The targeting and anchoring mechanisms of carrier proteins vary among the different surface proteins, and various approaches have been used to develop successful display systems.

As an example, the well-studied *Pseudomonas syringae* Ice Nucleation Proteins (INP) are frequently used as surface anchor for protein display in Gram-negative bacteria. C-terminal domain fusion with INP has been successfully used for cell surface display of several proteins, including levansucrase, carboxymethylcellulase (CMCase), salmobin, and organophosphorushydrolase (OPH). INP is an outer membrane protein found in *Erwinia, Pseudomonas* and *Xanthomonas* gender. It nucleates ice formation in supercooled water and causes frost injury to plants. Its internal repeats serve as templates for ice nucleation and the length of this region is adjustable inframe. Therefore, it is possible to display peptides or proteins using INP motifs of different lengths in order to prevent the potential steric hindrance of the desired surface displayed proteins (passenger). Indeed, whereas most cell-surface display systems are limited by the size of exogenous passenger protein, the INP-based system is able to display passenger domains as large as 60 kDa.

Despite the evolution of engineering in microbial cell surface display of proteins, there still remains a need to improve the level of proteins fusion exposure at the cell surface. In particular, there remains a need for developing new functionalized microbial cell surfaces with versatile functionalities and applications, such as biocatalyst or even live vaccines, and with high efficiency.

### SUMMARY OF THE INVENTION

In accordance with a first aspect, the invention relates to a recombinant modified *Gammaproteobacteriaceae* that displays on its cell surface a modified Curli system comprising:
i) a CsgA protein or a CsgA homologous protein linked to the surface of said *Gammaproteobacteriaceae;* and
ii) a scaffoldin comprising at least one cellulosomal component, said scaffoldin being fused to the C-terminus of the CsgA protein.

Advantageously, - the modified Curli system is endogenous or exogenous, or a combination of endogenous and exogenous components; - the *Gammaproteobacteriaceae* is selected from the group consisting of *Escherichia in particular E. coli, Enterobacter in particular Enterobacter sp., Salmonella, in particular Salmonella typhimurium and S. enterica Typhi str., Sodalis in particular Sodalis glossinidius, Pectobacterium in particular Pectobacterium atrosepticum, Photorhabdus in particular Photorhabdus luminescens, Serratia in particular Serratia proteamaculans, Yersinia in particular Yersinia pestis, and Buchnera in particular Buchnera aphidiola str,* Vibrio in particular *Vibrio* sp., Shewanella in particular *Shewanella* sp., Pseudomonas in particular *Pseudomonas* sp.; - the *Gammaproteobacteriaceae* is *E. coli;* - the at least one cellulosomal component is a carbohydrate binding module (CBM), a cohesin domain, a dockerin domain, said module or domains originating from any cellulosome-producing microorganism; - the at least one cellulosomal component comprises a CBM; - the at least one cellulosomal component comprises a cohesin domain, said cohesin domain being selected from the group consisting of a cohesin domain from *Clostridium (Ruminiclostridium) cellulolyticum* (also named *C. cellulolyticum*), a cohesin domain from *Clostridium (Ruminiclostridium) thermocellum* (also named *C. thermocellum),* a cohesin domain from *Ruminococcus flavefaciens* (also named *R. flavefaciens*)*,* and any cohesin domain from any cellulosome-producing microorganism; - the scaffoldin comprises any combination of the modules or domains as listed above; - the at least one cellulosomal component comprises a dockerin domain, said dockerin domain being selected from the group consisting of a dockerin domains from *C*. *cellulolyticum,* a dockerin domain from *C. thermocellum,* a dockerin domain from *R. flavefaciens,* and any dockerin domain from any cellulosome-producing microorganism; the scaffoldin comprises at least one cohesin domain and a CBM; - the cohesin domain is tightly bound to at least one protein harboring a dockerin fused to at least one cellulolytic enzyme, and wherein the dockerin is anchored to said cohesin domain; - the protein harboring the dockerin further comprises a CBM; and - the dockerin is selected from the group consisting of dockerin domain from *C. cellulolyticum,* dockerin domain from *C. thermocellum,* dockerin domain *from R. flavefaciens,* and any dockerin from any cellulosome-producing microorganism.

According to a second aspect, the invention concerns a use of the recombinant modified *Gammaproteobacteriaceae* as described above, for degrading lignocellulosic biomass.

According to third aspect, the invention relates to a use of the recombinant modified *Gammaproteobacteriaceae* as described above, as a biocatalyst.

According to fourth aspect, the invention relates to a use of the recombinant modified *Gammaproteobacteriaceae* as described above, as a cellular vector for vaccination.

According to a fifth aspect, the invention relates to a use of the recombinant modified *Gammaproteobacteriaceae* as described above, for bioremediation of toxic compounds or metals.

### BRIEF DESCRIPTION OF DRAWINGS

Other features and aspects of the present invention will be apparent from the following description and the accompanying drawings, in which:
Fig. 1A is a schematic representation of the hybrid scaffoldin Scaf6;
Fig. 1B is a schematic representation of the complex obtained when the Scaf6 is mixed with equimolar amounts of three chimeric cellulases;
Fig. 1C is a schematic representation of the complex obtained when the Scaf6 is mixed with equimolar amounts of three labelled chimeric cellulases;
Fig. 1D corresponds to the legend of the various elements shown in Fig. 1A to 1C;
Fig. 2A illustrates the complexation of Scaf6 with the labeled and unlabelled chimeric cellulases analyzed by non-denaturating polyacrylamide gel electrophoresis (PAGE); Arrow designates the band corresponding to the complex containing the three labelled cellulases.
Fig. 2B corresponds to the fluorescence imaging of the PAGE illustrated in Fig. 2A; Arrows designate the band corresponding to the complex containing the three labelled cellulases.
Fig. 2C illustrates the complexation of Scaf6 with the labelled chimeric cellulases by analyzing the sequential complexation of Scaf6 with labelled Cel48Ft, labelled Cel9Gc, and labelled Cel5Af by the sequential increment of band shifts analyzed by non-denaturing PAGE;
Fig. 3A is a diagram of *E.coli* bacteria outer membrane;
Fig. 3B is a diagram of the outer membrane of *E.coli* bacteria transformed with the plasmid encoding CsgA;
Fig. 3C is a diagram of the outer membrane of *E.coli* bacteria transformed with the plasmid encoding CsgA-Scaf6;
Fig. 3D shows from top to bottom the visible and fluorescence (triangle, DyeLight 488; circle, AlexaFluor 350 and star, Alexafluor 594) micrographs of *E.coli* bacteria shown in the diagram of Fig. 3A;
Fig. 3E shows from top to bottom the visible and fluorescence (triangle, DyeLight 488; circle, AlexaFluor 350 and star, Alexafluor 594) micrographs of *E.coli* bacteria transformed with the plasmid encoding CsgA shown in the diagram of Fig. 3B;
Fig. 3F shows from top to bottom the visible and fluorescence (triangle, DyeLight 488; circle, AlexaFluor 350 and star, Alexafluor 594) micrographs of *E.coli* bacteria transformed with the plasmid encoding CsgA-Scaf6 shown in the diagram of Fig. 3C;
Fig. 4A is a diagram of the outer membrane of *E.coli* bacteria incubated with labelled chimeric cellulases Cel48Ft, Cel9Gc and Cel5Af;
Fig. 4B is a diagram of the outer membrane of *E.coli* bacteria transformed with the plasmid encoding CsgA and incubated with labelled chimeric cellulases Cel48Ft, Cel9Gc and Cel5Af;
Fig. 4C is a diagram of the outer membrane of *E.coli* bacteria outer membrane transformed with the plasmid encoding CsgA-Scaf6 and incubated with labelled chimeric cellulases Cel48Ft, Cel9Gc and Cel5Af. It illustrates the interaction between cell surface displayed CsgA-Scaf6 fusion and the labelled chimeric fluorescent cellulase probes;
Fig. 4D shows from top to bottom the visible and fluorescence (triangle, DyeLight 488; circle, AlexaFluor 350 and star, Alexafluor 594) micrographs of *E.coli* bacteria and incubated with labelled chimeric cellulases Cel48Ft, Cel9Gc and Cel5Af shown in the diagram of Fig. 4A;
Fig. 4E shows from top to bottom the visible and fluorescence (triangle, DyeLight 488; circle, AlexaFluor 350 and star, Alexafluor 594) micrographs of *E.coli* bacteria transformed with the plasmid encoding CsgA and incubated with labelled chimeric cellulases Cel48Ft, Cel9Gc and Cel5Af shown in the diagram of Fig. 4B; and
Fig. 4F shows from top to bottom the visible and fluorescence (triangle, DyeLight 488; circle, AlexaFluor 350 and star, Alexafluor 594) micrographs of *E.coli* bacteria transformed with the plasmid CsgA-Scaf6 and incubated with labelled chimeric cellulases Cel48Ft, Cel9Gc and Cel5Af shown in the diagram of Fig. 4D.

### DETAILLED DESCRIPTION OF THE INVENTION

The invention relates to recombinant modified microorganisms displaying membrane functionalization for various biotechnological applications and, in particular, to a recombinant modified *Gammaproteobacteriaceae* that displays, on its cell surface, a modified Curli system comprising a fusion protein derived from the Curli system. Curli-type systems are the major proteinaceous component of the Curli fibers which allow the formation of a complex extracellular matrix by many *Gammaproteobacteriaceae.* Curli-type fibers are involved in adhesion to surfaces, cell aggregation, and biofilm formation. Curli-type systems also mediate host cell adhesion and invasion. Curli-type systems belong to a growing class of fibers known as amyloids, which are aggregates of proteins that become folded into a shape that allows many copies of that protein to stick together, forming fibrils. As an example, in the *Enterobacteriaceae E. coli* the genes involved in the production of Curli form two adjacent operons encoding a transcriptional activator (*csgD*)*,* an outer membrane Curli-specific translocation channel component (*csgG*), a periplasmic chaperon (*csgE*), a protein of unknown function (csgF), as well as the major and minor structural subunits, CsgA and CsgB. CsgA is secreted through the outer membrane translocation channel as a monomer and its polymerization at the extracellular surface is nucleated by the minor structural protein CsgB. The capacity of the Curli system to be used as a surface anchor for exogenous protein (passenger) cell surface exposure in Gram-negative bacteria has been tested using a C-terminal heterologous protein fusion with CsgA (van Gerven N., Goyal P., Vandenbussche G., de Kerpel M., Jonckheere W., De Greve H., Remaut H. 2014. Secretion and functional display of fusion proteins through the curli biogenesis pathway. Mol. Microbiol. 9: 1022-1035). It was reported that the narrow transverse diameter of the translocation channel formed by CsgG (approximately 1 nm) cannot translocate larger proteins. Thus, displaying larger versatile proteins at the cell surface of the *Gammaproteobacteriaceae via* the Curli system appears to be a difficult approach.

However, according to the invention, the Curli pathway is challenged for cell surface exposure of rather large proteins, such as scaffoldins. Scaffoldins are essential components of extracellular large multienzymatic complexes called cellulosomes produced by most cellulolytic bacteria living in anaerobic biotopes. Cellulosomes produced by *C. cellulolyticum* (ATCC35319) are composed of a scaffoldin CipC containing a CBM and eight homologous receptor modules called cohesins. All the cellulases, hemicellulases and other plant cell wall modifying enzymes entering *C. cellulolyticum's* cellulosomes composition contain a dockerin module. This dockerin module can bind with a high affinity (K_{d} values ranging between 10⁻⁹ and 10⁻¹⁰ M in *C. cellulolyticum*) to each cohesin module of CipC. Nevertheless, within a given species and type the cohesin/dockerin interaction tends to be non-specific, i.e. an enzyme bearing its dockerin module can bind to any of the cognate cohesins displayed by the scaffoldin of the same species with similar affinity. In contrast, this strong protein/protein interaction is usually species-specific. For instance, the native dockerin harbored by a cellulosomal enzyme from *C. cellulolyticum* cannot bind to the cohesins borne by the major scaffoldin (CipA) of *C. thermocellum* and vice versa.

Hence, to develop new functionalized microbial cell surfaces for versatile and various applications, the inventors designed new artificial cellulosomes composed of a hybrid scaffoldin displaying cohesins from different bacterial species, and enzymes engineered to exhibit the cognate dockerins. In this purpose, the position of each enzyme onto the hybrid scaffoldin is strictly controlled thereby leading to a highly homogenous and organized complex of different known proteins, in contrast to natural cellulosomes which are heterogeneous in terms of enzymes composition, stoichiometry and location.

In this context, the recombinant modified *Gammaproteobacteriaceae* according to the invention comprises a CsgA protein tightly associated to the surface of said *Gammaproteobacteriaceae,* and a scaffoldin comprising at least one cohesin domain, said scaffoldin being fused to the C-terminus of the CsgA protein.

Preferably, the cohesin domain is selected from the group consisting of cohesin domain from *C*. *cellulolyticum* (ATCC35319), cohesin domain from *C*. *thermocellum* (ATCC27405), and cohesin domain from *R*. *flavefaciens* (ATCC2213). But specific cohesins from any other cellulosome-producing microorganism can be used. *C. cellulolyticum* is an anaerobic, motile, gram-positive bacterium. The catalytic components of this cellulosome release soluble cello-oligosaccharides from cellulose providing the primary carbon substrates to support bacterial growth. *C. thermocellum* is an anaerobic, thermophilic bacterium which is capable of directly converting a cellulosic substrate into ethanol. *R. flavefaciens* is an anaerobic, cellulolytic bacterium found in the rumen and in the hindgut of monogastric domestic and wild mammals. These and other cellulolytic bacteria play an important role in the digestion of hemicellulose and cellulose from plant cell walls.

According to a preferred embodiment of the invention, the scaffoldin further comprises a CBM interacting with carbohydrate substrates (polysaccharides) from the plant cell wall or derived from its degradation, or polysaccharides of other sources (chitin, glycogen, etc). They are classified into numerous families (around 64 families), based, in particular, on amino acid sequence similarity. As an example, family 3a carbohydrate-binding modules (CBM3a) have a β-sandwich structure, could be associated with a wide range of bacterial glycosyl hydrolases that would allow to decrease the distance between the catalytic domain of said hydrolases from their substrates, and is involved in cellulose binding.

According to a preferred embodiment of the invention, the scaffoldin comprises three cohesin domains and a CBM.

In another preferred embodiment, the cohesin domain anchors at least one protein comprising a dockerin domain fused to at least one cellulolytic enzyme. In a preferred mode for carrying out the invention, the protein comprising the dockerin further comprises a catalytic domain and optional CBM.

According to a preferred embodiment of the invention, the dockerin is selected from the group consisting of dockerin domain from *C*. *cellulolyticum,* dockerin domain from *C. thermocellum,* and dockerin domain from *R. flavefaciens,* or from any cellulosome-producing microorganism.

In a preferred embodiment of the invention, the *Gammaproteobacteriaceae* is selected from the group consisting of *E. coli, Enterobacter sp., Salmonella typhimurium, S. enterica Typhi str., Sodalis glossinidius, Pectobacterium atrosepticum, Photorhabdus luminescens, Serratia proteamaculans, Yersinia pestis, and Buchnera aphidiola str..* Even more preferably, the *Gammaproteobacteriaceae* is *Escherichia coli,* also named *E. coli.*

According to a preferred embodiment of the invention, the Curli system in the recombinant modified *Gammaproteobacteriaceae* is endogenous or exogenous.

As used herein, the term "endogenous" means a material that is naturally produced by a mammal, an organism, a tissue or a cell. In other words, "endogenous substances/processes" mean that the substances and the processes are those that originate from within an organism, tissue or cell. In particular, the terms "endogenous Curli system" mean that the Curli system originate from within an organism, tissue or cell. In contrast, the terms "non-endogenous" or "exogenous" in this context qualified material which is not naturally produced by a mammal, an organism. In other words, the terms "exogenous substances/processes" mean that substances/processes are introduced into the mammal, the organism, the tissue or the cell. The terms "introduced into" mean that the ordinary skill in the art makes a genetic or other manipulations of the organism.

In a second aspect, the invention concerns a use of the recombinant modified *Gammaproteobacteriaceae* as above described, for degrading cellulose biomass, by anchoring cellulases and/or hemicellulases appended with appropriate dockerin modules onto the exposed scaffoldin, and incubation of the modified *Enterobacteriaceae* with cellulosic material to induce the release of fermentable mono- and oligosaccharides.

In a third aspect, the invention relates to a use of the recombinant modified *Gammaproteobacteriaceae* as above described, as a biocatalyst. For instance, a glucokinase, a phosphoglucomutase and a phosphofructokinase can be grafted with appropriate dockerin modules and anchored onto the scaffoldin exposed by the modified *Gammaproteobacteriaceae* as to produce from glucose and ATP, large amounts of fructose-1,6-biphosphate at low cost.

In a fourth aspect, the invention relates to a use of the recombinant modified *Gammaproteobacteriaceae* as above described, as a live vaccines, by grafting dockerin modules to three different proteinaceous antigens (or three different variants of the same antigen like hemagglutinine and neuraminidase for instance in the case of the influenza viruses) of the targeted pathogen and anchoring them onto the scaffoldin exposed on the cell surface of the recombinant modified non-pathogenic *Gammaproteobacteriaceae,* prior injection to the subject for immunization.

In a fifth aspect, the invention relates to a use of the recombinant modified *Gammaproteobacteriaceae* as above described, for bioremediation of toxic compounds/metals. Arsenite reductase, chromate reductase, nitrate reductase, sulfite reductase etc could be modified to bear a suitable dockerin module and bound onto the scaffoldin exposed by the recombinant modified *Gammaproteobacteriaceae.* The recombinant bacterium could then be used for depollution of (multiple)toxic-contaminated fluids.

### MATERIALS, METHODS AND EXAMPLES

### 1.Materials and methods.

### 1.1. Strains and plasmids

The pET28* vector (Novagen) is generated by replacement of the ATA**CCATGG** sequence (NcoI restriction site in bold) by the **GGATCC**ATGA sequence (BamHI restriction site in bold). The pET9dScaf6 without BamHI restriction site in the Scaf6 insert (scaf6 - BamHI) is generated by site-directed mutagenesis using the primers MutBamHIScaf6up (GTTGGAGATATA**GGATCA**GCCGGTGGTTTAT, mutated BamHI restriction site in bold) and MutBamHIScaf6do (ATAAACCACCGGC**TGATCC**TATATCTCCAAC, mutated BamHI restriction site in bold) and the vector pET9dScaf6H (H.-P. Fierobe, F. Mingardon, A. Mechaly, A. Belaich, MT Rincon, S. Pages, C. Tardif, R. Lamed, J.-P. Belaich and E. A. Bayer. 2005. Action of designer cellulosomes on homogeneous versus complex substrates: controlled incorporation of three distinct enzymes into a defined trifunctional scaffoldin. J. Biol. Chem. 280 : 16325-34) as template. The resulting plasmid is then used as template to amplify the "scaf6 - BamHI" amplicon using the primers NheIScaf6up (ggggg**GCTAGC**GGCGATTCTCTTAAAGTTACAG, NheI restriction site in bold) and XhoIScaf6do (ggggg**CTCGAG**CTTAACAATGATAGCGCCATCAG, XhoI restriction site in bold). The CsgA amplicon is produced by PCR using the primers BamHICsgAup (ggggg**GGATCC**ATGAAACTTTTAAAAGTAGCAGC, BamHI restriction site in bold) and NheICsgAdo (ggggg**GCTAGC**GTACTGATGAGCGGTCGCGT, NheI restriction site in bold) and the genomic DNA from *E. coli* MG1655 as template. The CsgA and "scaf6 - BamHI" amplicons and the pET28* are digested with the appropriate restriction enzymes. The fragments of interest are purified and the BamHI-XhoI pET28* fragment is subsequently dephosphorylated. The plasmid pET28CsgAScaf6 coding for the chimeric enzyme CsgA-Scaf6 is constructed by ligation of the dephosphorylated BamHI-XhoI pET28* fragment with the BamHI-NheI CsgA and the NheI-XhoI "scaf6 - BamHI" fragments. To construct the plasmid pET28CsgA coding for the native CsgA protein, the overlapping oligomers DBstopcarrierNheI-XhoIup (**CTAGC**TGATGACCGCGGC, NheI overhang fragment in bold, overlapping sequence underligned) and DBstopcarrierNheI-XhoIdo (**TCGAG**CCGCGGTCATCAG, XhoI overhang fragment in bold, overlapping sequence underligned) are annealed to generate the DBstopcarrierNheI-XhoI fragment containing two stop codons in series (TGATGA) and the overhangs extremities normally generated by the NheI and XhoI enzyme digestion. pET28CsgA is obtained by ligation of the annealed DBstopcarrierNheI-XhoI fragment with the BamHI-NheI CsgA and the dephosphorylated BamHI-XhoI pET28* fragments. The NEB5α *E.coli* strain (Biolabs) is used as the host strain for cloning and positive clones are verified by DNA sequencing. The MG1655 (DE3) *E. coli* strain, which was obtained by using the λDE3 Lysogenization Kit (Novagen), is used as expression strain.

### 1.2. Protein expression and purification, CsgA and CsgA-Scaf6 expression.

The chimeric proteins (cellulases coupled with species specific dockerins) are produced and purified as formerly reported (Fierobe H.-P., Mechaly A., Tardif C., Belaich A., Lamed R., Shoham Y., Belaich J.-P., and Bayer E. A.. 2001. Design and production of active cellulosome chimeras: Selective incorporation of dockerin-containing enzymes into defined functional complexes. J. Biol. Chem. 276: 21257-21261; Fierobe H.-P., Mingardon F., Mechaly A., Belaich A., Rincon MT, Pages S., Tardif C., Lamed R., Belaich J.-P. and Bayer E. A. 2005. Action of designer cellulosomes on homogeneous versus complex substrates: controlled incorporation of three distinct enzymes into a defined trifunctional scaffoldin. J. Biol. Chem. 280 : 16325-34): Cel48Ft (cellulase Cel48F coupled with the *C. thermocellum* dockerin), Cel5Af (cellulase Cel5A coupled with the *R. flavefaciens* dockerin) and Cel9Gc (cellulase Cel9G coupled with the *C. cellulolyticum* dockerin). The MG1655 (DE3) *E. coli* strains overproducing CsgA and CsgAScaf6 are grown in 250-mL erlenmeyer flasks at 37°C in 50 mL Lysogeny Broth supplemented with 50 µg/mL kanamycin at 150 rpm until A₆₀₀ = 0.7-0.9. The induction of the expression is performed for 24 h at 37°C with 500 µM isopropyl thio-β-D-galactoside (IPTG). At the end of the induction phase, an equivalent of 100µL of cells at A₆₀₀ = 4.5 is harvested by centrifugation (8000 *g*, 5 min at 4°C) and resuspended in 500 µL of cold 10 mM Tris-HCl pH 8.0, 1 mM CaCl₂, 200 mM NaCl. A control is performed in the same conditions using a MG1655 (DE3) *E. coli* strain untransformed without antibiotics.

The DNA and protein sequences of CsgA, CsgA-Scaf6, Cel48Ft, Cel9Gc and Cel5Af are provided in an appendix of this description.

### 1.3. Protein labelling, determination of the labelling degree and in vitro complexation.

Cel48Ft, Cel5Af and Cel9Gc are concentrated and buffer exchanged by ultrafiltration (3 times) with cold 20 mM potassium phosphate pH 8. The concentration of the proteins is estimated by absorbance at 280 nm in this buffer using the program ProtParam tool (https://web.expasy.org/protparam/). Proteins are labelled in the dark following suppliers' protocols: Cel48Ft and Cel9Gc with Alexa Fluor 350 protein Labeling kit (Thermo Fischer Scientific, excitation wavelength [Ex] = 346nm, emission wavelength [Em] = 442 nm) and Alexa Fluor 594 protein Labeling kit (Thermo Fischer Scientific, Ex = 590 nm, Em = 617 nm) respectively, and Cel5Af with DyLight 488 NHS Ester (Thermo Fisher Scientific, Ex = 493 nm, Em = 518 nm). Labelled proteins are buffer exchanged by ultrafiltration (5 times) in the dark with cold 10 mM Tris-HCl pH 8, 1 mM CaCl₂ in order to remove dyes in excess. The determination of the labelling degree is performed according to suppliers' protocols. *In vitro* complexation between Scaf6 and labelled or unlabelled Cel48Ft, Cel5Af and Cel9Gc are performed as previously described (Borne R., Bayer E. A., Pages S., Perret S., Fierobe H.-P. (2013) Unraveling enzyme discrimination during cellulosome assembly independent of cohesin/dockerin affinity. FEBS J. 280:5764-79): samples (10 µM final concentration) are mixed and incubated 5 minutes at room temperature in 20 mM Tris/maleate (pH 6.0) and 1 mM CaCl₂ and 4 µL of each mix is subjected to non-denaturing polyacrylamide gel electrophoresis (PAGE, 4-15% gradient gel) with a Phastsystem apparatus (GE Healthcare). Complexation is checked by Coomassie blue staining or Fluorescence scanning using suitable filter with a Typhoon FLA 9500 (GE Healthcare).

### 1.4. In vivo complexation, UV-visible microscopy and fluorescence microscopy.

For *in vivo* complexation, bacteria overproducing (or not) CsgA and CsgAScaf6 are grown and induced as described above. Cells (equivalent of 100µl of cells at A600 = 4.5) are resuspended in 500µl of cold 10 mM Tris-HCl pH 8.0, 1 mM CaCl2, 200mM NaCl (see above). 50µl of resuspension are loaded into the poly-L-lysine treated channels of IBIDI flow chambers µ-Slides (1µ-slide VI0.4 Poly-L-Lysine, IBIDI) and incubated for 30 minutes at room temperature. Fixed cells are washed 3 times as described by the supplier with 100µl of cold 10 mM Tris-HCl pH 8.0, 1 mM CaCl₂, 200mM NaCl. After removing all liquid, 50 to 100µL of labelled proteins (10µM final concentration for each) are applied on the fixed cells and incubated for 30 min at room temperature in the dark. The excess labelling is removed by 3 times washing with 100µl of cold 10 mM Tris-HCl pH 8.0, 1 mM CaCl₂, 200mM NaCl and the stained µ-Slides are kept in the dark. For the imaging, slides are examined by microscopy using a Nikon Eclipse TE2000 E PFS inverted epifluorescence microscope with a 100 x magnification oil immersion lens (objective NA 1.3 Phase Contrast). *E. coli* cells UV-visible and Fluorescence micrographs are acquired in the visible range and using the appropriate filters and settings for the EPI fluorescence microscopy: GFP (for labelled Cel5Af), DAPI (for labelled Cel48Ft) and Texas Red (for labelled Cel9Gc) . Manual image analysis and comparison are processed using the Fiji/ImageJ package.

### 2. Examples

### 2.1. In vitro assembly of trivalent heterogeneous minicellulosomes and fluorescence labelling.

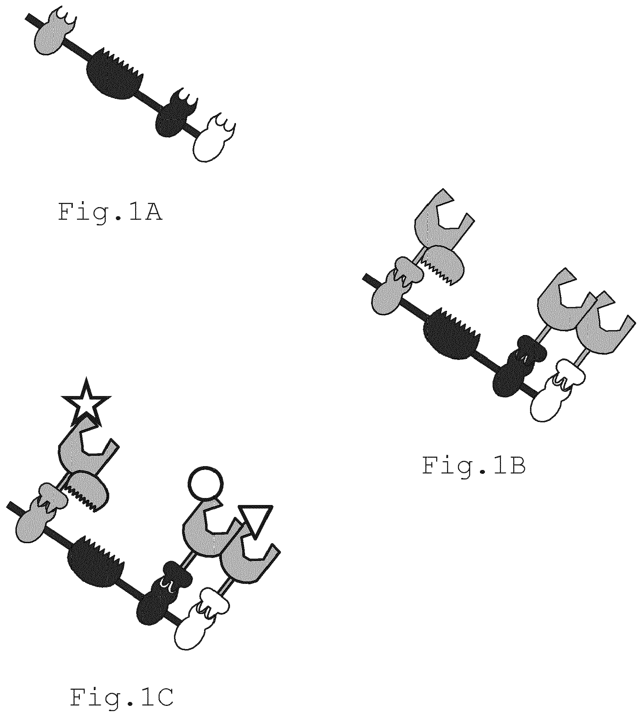

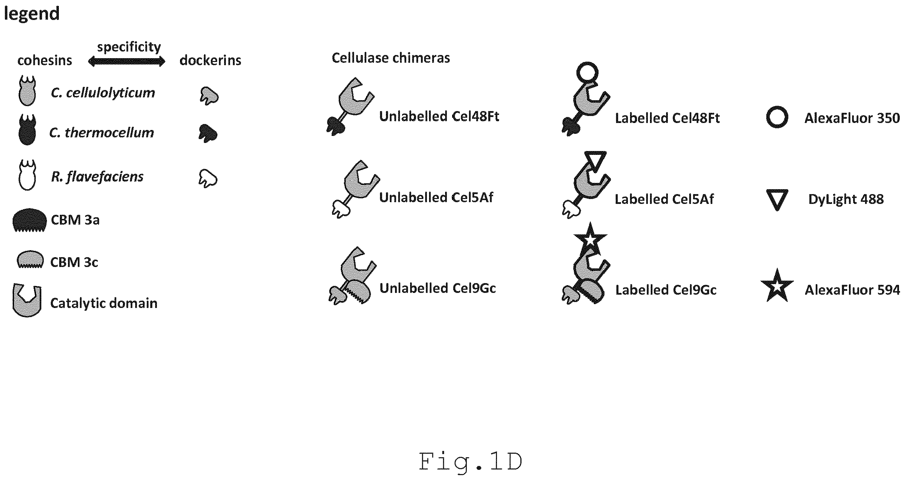

The hybrid scaffoldin, named Scaf6, contains a family 3a CBM (CBM3a) framed by one cohesin module from *C. cellulolyticum* at the N-terminus, and two cohesin modules from *C. thermocellum* and *R. flavefaciens,* respectively, at the C-terminus (Fig. 1A). When mixed with equimolar amounts of Cel48F from *C. cellulolyticum* with a *C. thermocellum* dockerin module appended (Cel48Ft), a Cel9G from *C. cellulolyticum* with a *C. cellulolyticum* dockerin module appended (Cel9Gc) and a Cel5A from *C. cellulolyticum* with a *R. flavefaciens* dockerin module appended (Cel5Af), this hybrid scaffoldin generates with the chimeric cellulase a single and well-ordered complex with a known enzymatic composition (Fig. 1B).

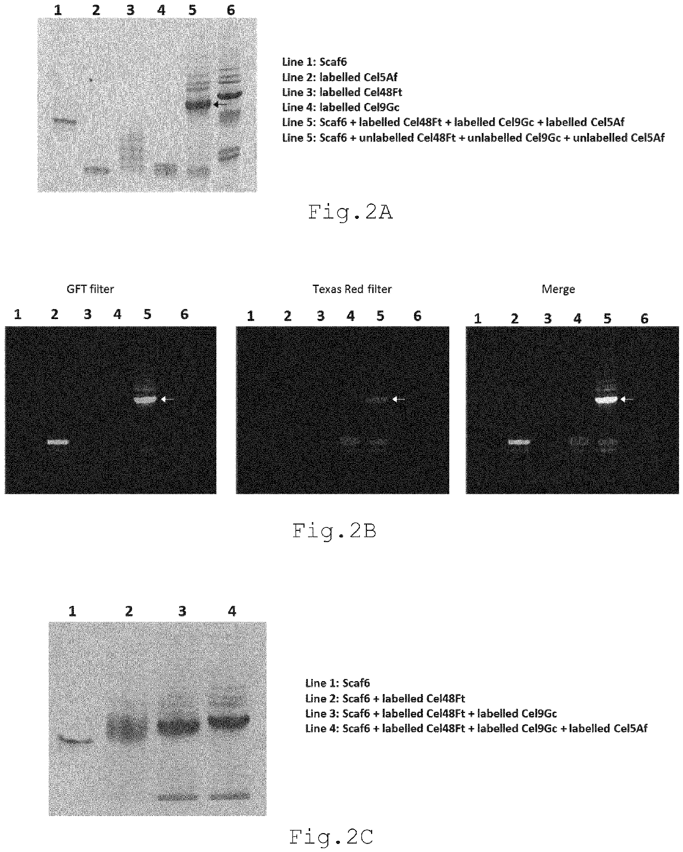

To evaluate the effect of the specific grafting of a fluorescent molecular probes to each chimeric cellulase on the formation of the complex *in vitro* (Fig. 1C, Table 1), the complexation of Scaf6 with the labelled and unlabelled chimeric cellulases is verified by non-denaturing PAGE (Fig. 2).

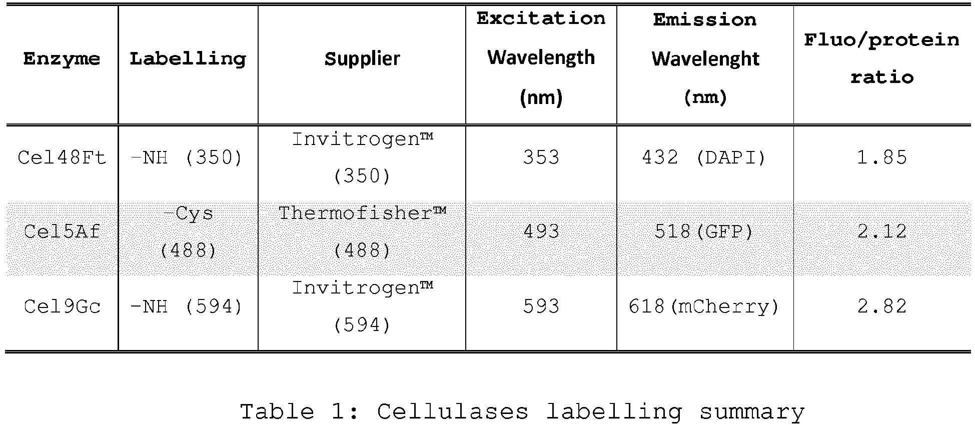

The Coomassie blue stained gel shows that the addition of the labelled proteins (Cel48Ft, Cel5Af and Cel9Gc) to the Scaf6 in an equimolar ratio induced in native condition a migration shift (Fig. 2A, lines 1 and 5) slightly different from the one observed for the mix between the Scaf6 and the unlabelled proteins (Cel48Ft, Cel5Af and Cel9Gc, Fig. 2A, line 6). Moreover, the fluorescence imaging (Fig. 2B) demonstrates that the complex formed upon addition of the labelled proteins contains at least Scaf6, Cel5Af and Cel9Gc (Fig. 2A, lines 2, 4 and 5). The formation of the full complex is confirmed by analyzing the sequential complexation of Scaf6 with Cel48Ft, Cel9Gc and Cel5Af which is reflected by the sequential increments of band shifts on non-denaturing PAGE (Fig. 2C) .

These results demonstrate a complexation *in vitro* of the labelled proteins with the Scaf6 and that the labelling does not affect the formation of a single and well-ordered complex with a known enzymatic composition. Nevertheless the observed band shift difference in the Fig.2A can rise from the mass difference induced by the labelling of the interacting proteins. Thus, altogether, these observations *in vitro* confirm that labelled Cel48Ft, Cel5Af and Cel9Gc can be used as fluorescent probes to test the cell surface exposure of the CsgA-Scaf6 chimera.

### 2.2. In vivo assembly and cell surface exposition of trivalent heterogeneous minicellulosomes.

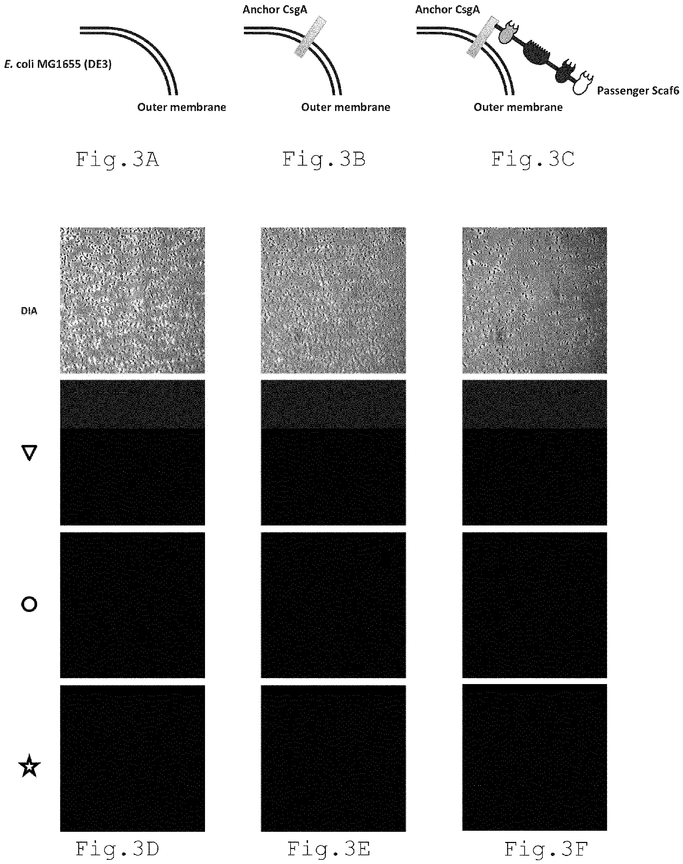

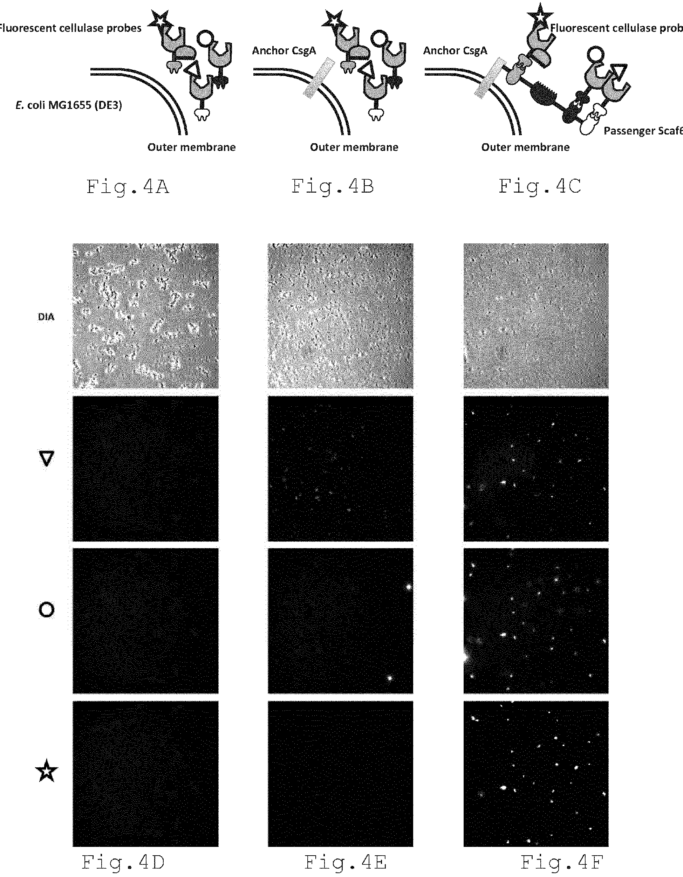

It is then examined whether the CsgA-Scaf6 (CsgA anchor fused to Scaf 6 passenger protein) can attain the cell surface in its native fold and consequently be exposed and functional (Fig.3 and Fig.4). The functionality of the three cohesin modules from different species origins that the CsgA-Scaf6 displays is tested to confirm the CsgA-Scaf6 structural conformation integrity and its exposition on the bacterial cell surface (Fig.3C). As matter of fact, the species specific cohesin-dockerin interactions should, as described above *in vitro,* allow the recruitment of labelled Cel48Ft, Cel5Af and Cel9Gc by the Scaf6 passenger of CsgA-Scaf6 proteins exposed on the cell surface (Fig.4C), thereby leading to fluorescence emission. To be able to use this reporter method, it is also tested this fluorescent labelling *in vivo* on cell overexpressing or not CsgA (Fig.3A/3B/4A and 4C).

After a 24h induction, visible and fluorescence micrographs of bacteria producing the CsgA-Scaf6 fusion protein without the three fluorescent probes revealed no significant auto-fluorescence (Fig.3F), as for the bacteria transformed or not with a plasmid encoding CsgA (Fig.3D and 3E). Contrary to the MG1655 (DE3) *E. coli* cells and the one harbouring the plasmid pET28CsgA (Fig.4D and 4E respectively), bacteria harbouring the plasmid pET28CsgAScaf6 showed after incubation with labelled Cel48Ft, labelled Cel5Af and labelled Cel9Gc, a mark fluorescence pattern represented by white spots in the Fig.4F. The matching between the whites spots in fluorescence micrographs (Fig.4F) supports that the marked cells exposed on their surface the three fluorescent probes (Fig.4F). Moreover, no fluorescence is detected for cells expressing or not CsgA alone (Fig.4D and 4E) suggesting that no non-specific interactions occurred between the fluorescent cellulases and the cells with or without CsgA surface exposed. It is thus concluded that the probes recruitment at the surface of the CsgA-Scaf6 expressing cells arise from specific interactions between this fusion-protein and the labelled cellulases. This is also supported by the presence in the Fig.4F of spots that are shown in said figure corresponding to the individual, dual or triple recruitment of the labelled cellulases.

However, not all the cells expressing CsgA-Scaf6 are labelled (Fig.4F) but this phenomenon can be correlated with the use of IPTG to trigger the production of the fusion protein. It has been reported that IPTG heterogeneously induces the expression of a gene under the control of a lac (or lac-derivative) promoter in a cell population (Portle S. Causey T.B., Wolf K., Bennett G.N. San K.Y. Mantzaris N. 2007. Cell population heterogeneity in expression of a gene-switching network with fluorescent markers of different half-live J. Biotechnol. 128: 362-75. Wu F., Su R.Q. Lai Y.C., Wang X. 2047. Engineering of a synthetic quadrastable gene network to approach Waddington landscape and cell fate determination. Elife e23702). Moreover, the wild type or CsgA-fusion producing bacteria (CsgA bacteria) exhibit a modified morphology compared to MG1655 (DE3) *E. coli* cells (Fig.3 and Fig.4). This phenotype can be linked to the initial function of CsgA in *Gammaproteobacteriaceae* which is the major component of Curli amyloids fibers produced in response to stressful environmental conditions and constituting an extracellular protection associated to the cell surface.

Altogether, these data demonstrate that CsgA-Scaf6 protein is produced, secreted, addressed and exposed to the *E. coli* cell surface. This result is surprising since the Type VIII secretion pathway implicated in the Curli formation is mediated by a CsgG transmembrane pore whose diameter appears to control the transport efficiency of the CsgA-fusion. It has been proposed that the diameter size of the introduced passenger polypeptides in its folded conformation should not exceed roughly 1 nm to be translocated across the outer membrane (P. Goyal, P. V. Krasteva, N. van Gerven, F. Gubellini, I. van den Broeck, A. Troupiotis-Tsaïlaki, W. Jonckheere, G. Péhau-Arnaudet, J. S. Pinkner, M. R. Chapman, S. J. Hultgren, S. Howorka, R. Fronzes and H. Remaut. Structural and mechanistic insights into the bacterial amyloid secretion channel CsgG. 2014. Nature : 516 (7530) : 250-253). Despite a molecular weight of 86.8 kDa for the CsgA-Scaf6 fusion (15 kDa for CsgA + 71.5 kDa for Scaf6), the chaplet structure of the Scaf6 moiety is efficiently secreted. In particular, the Scaf6 component adopts a suitable conformation allowing its secretion in fusion with CsgA via the natural *E. coli* Type VIII secretion pathway, and is probably integrated into the extracellular fibers generated by CsgA polymerization. Moreover, each specific cohesin module of the Scaf6 passenger is likely to attain its native fold since they are able to specifically interact with the corresponding dockerin module fused to fluorescent labelled cellulases.

Since the fusion protein is surface-exposed via the CsgA domain and is fully functional via the Scaf6 multi-domains part, the inventors have developed a system able to functionalize at will the *E. coli* membrane. As a matter of fact, this surface exposed material derived from the natural Curli system of *E*. *coli* allows the recruitment of the desired proteins and to spatially regroup enzymes working in tandem in a designed complex in order to increase the efficiency of the global catalyzed reaction. This is achieved by fusing the corresponding dockerin module to the desired protein herein the cellulases used in accordance with the invention.

### Conclusion

According to the invention, it has been developed a novel strain of non-pathogenic *E. coli* which massively displays at the cell surface a hybrid scaffoldin whose three cohesins can robustly anchor three proteins appended with the corresponding dockerin modules. This new highly flexible tool can be exploited in a large variety of biotechnological applications. For instance, the engineered *E. coli* cells can host at least three enzymes involved in three successive steps in a specific pathway and form a biocatalyst that after desired product synthesis, can be easily removed from the sample. Bioremediation using appropriate proteins to capture/oxidize the toxic compound or living vaccines exhibiting three different antigens of the same pathogenic microbe on the cell surface exposed Scaf6 can also be planned. Moreover, this synthetic system can be improved by the addition of other cohesin domains to enlarge the partners combinations. Natural cellulosomes can display more than 10 cohesins on a scaffoldin, whereas synthetic cellulosomes based on 8 different specific cohesin/dockerin systems were successfully assembled *in vitro.*

### APPENDIX RELATING TO SEQUENCES

>CsgA DNA 
ATGAAACTTTTAAAAGTAGCAGCAATTGCAGCAATCGTATTCTCCGGTAGCG
CTCTGGCAGGTGTTGTTCCTCAGTACGGCGGCGGTAACCACGGTGGTGG
CGGTAATAATAGCGGCCCAAATTCTGAGCTGAACATTTACCAGTACGGTGGC
GGTAACTCTGCACTTGCTCTGCAAACTGATGCCCGTAACTCTGACTTGACTA
TTACCCAGCATGGCGGCGGTAATGGTGCAGATGTTGGTCAGGGCTCAGATGA
CAGCTCAATCGATCTGACCCAACGTGGCTTCGGTAACAGCGCTACTCTTGAT
CAGTGGAACGGCAAAAATTCTGAAATGACGGTTAAACAGTTCGGTGGTGGCA
ACGGTGCTGCAGTTGACCAGACTGCATCTAACTCCTCCGTCAACGTGACTCA
GGTTGGCTTTGGTAACAACGCGACCGCTCATCAGTACGCTAGCTGA
>CsgA protein 
MKLLKVAAIAAIVFSGSALAGVVPQYGGGNHGGGNNSGPNSELNIYQYGG
GNSALALQTDARNSDLTITQHGGGNGADVGQGSDDSSIDLTQRGFGNSATLD
QWNGKNSEMTVKQFGGGNGAAVDQTASNSSVNVTQVGFGNNATAHQYAS-
>CsgA-Scaf6 DNA 
ATGAAACTTTTAAAAGTAGCAGCAATTGCAGCAATCGTATTCTCCGGTAGCG
CTCTGGCAGGTGTTGTTCCTCAGTACGGCGGCGGTAACCACGGTGGTGG
CGGTAATAATAGCGGCCCAAATTCTGAGCTGAACATTTACCAGTACGGTGGC
GGTAACTCTGCACTTGCTCTGCAAACTGATGCCCGTAACTCTGACTTGACTA
TTACCCAGCATGGCGGCGGTAATGGTGCAGATGTTGGTCAGGGCTCAGATGA
CAGCTCAATCGATCTGACCCAACGTGGCTTCGGTAACAGCGCTACTCTTGAT
CAGTGGAACGGCAAAAATTCTGAAATGACGGTTAAACAGTTCGGTGGTGGCA
ACGGTGCTGCAGTTGACCAGACTGCATCTAACTCCTCCGTCAACGTGACTCA
GGTTGGCTTTGGTAACAACGCGACCGCTCATCAGTACGCTAGCGGCGATTCT
CTTAAAGTTACAGTAGGAACAGCTAATGGTAAGCCTGGCGATACAGTAACAG
TTCCTGTTACATTTGCTGATGTAGCAAAGATGAAAAACGTAGGAACATGTAA
TTTCTATCTTGGATATGATGCAAGCCTGTTAGAGGTAGTATCAGTAGATGCA
GGTCCAATAGTTAAGAATGCAGCAGTTAACTTCTCAAGCAGTGCAAGCAACG
GAACAATCAGCTTCCTGTTCTTGGATAACACAATTACAGACGAATTGATAAC
TGCAGACGGTGTGTTTGCAAATATTAAGTTCAAATTAAAGAGTGTAACGGCT
AAAACTACAACACCAGTAACATTTAAAGATGGTGGAGCTTTTGGTGACGGAA
CTATGTCAAAGATAGCTTCAGTTACTAAGACAAACGGTAGTGTAACGATCGA
TCCGACCAAGGGAGCAACACCAACAAATACAGCTACGCCGACAAAATCAGCT
ACGGCTACGCCCACCAGGCCATCGGTACCGACAAACACACCGACAAACACAC
CGGCAAATACACCGGTATCAGGCAATTTGAAGGTTGAATTCTACAACAGCAA
TCCTTCAGATACTACTAACTCAATCAATCCTCAGTTCAAGGTTACTAATACC
GGAAGCAGTGCAATTGATTTGTCCAAACTCACATTGAGATATTATTATACAG
TAGACGGACAGAAAGATCAGACCTTCTGGTGTGACCATGCTGCAATAATCGG
CAGTAACGGCAGCTACAACGGAATTACTTCAAATGTAAAAGGAACATTTGTA
AAAATGAGTTCCTCAACAAATAACGCAGACACCTACCTTGAAATAAGCTTTA
 
CAGGCGGAACTCTTGAACCGGGTGCACATGTTCAGATACAAGGTAGATTTGC
AAAGAATGACTGGAGTAACTATACACAGTCAAATGACTACTCATTCAAGTCT
GCTTCACAGTTTGTTGAATGGGATCAGGTAACAGCATACTTGAACGGTGTTC
TTGTATGGGGTAAAGAACCCGGTGGCAGTGTAGTACCATCAACACAGCCTGT
AACAACACCACCTGCAACAACAAAACCACCTGCAACAACAATACCGCCGTCA
GATGATCCGAATGCAATAAAGATTAAGGTGGACACAGTAAATGCAAAACCGG
GAGACACAGTAAATATACCTGTAAGATTCAGTGGTATACCATCCAAGGGAAT
AGCAAACTGTGACTTTGTATACAGCTATGACCCGAATGTACTTGAGATAATA
GAGATAAAACCGGGAGAATTGATAGTTGACCCGAATCCTGACAAGAGCTTTG
ATACTGCAGTATATCCTGACAGAAAGATAATAGTATTCCTGTTTGCAGAAGA
CAGCGGAACAGGAGCGTATGCAATAACTAAAGACGGAGTATTTGCTACGATA
GTAGCGAAAGTAAAATCCGGAGCACCTAACGGACTCAGTGTAATCAAATTTG
TAGAAGTAGGCGGATTTGCGAACAATGACCTTGTAGAACAGAGGACACAGTT
CTTTGACGGTGGAGTAAATGTTGGAGATATAGGATCAGCCGGTGGTTTATCC
GCTGTGCAGCCTAATGTTAGTTTAGGCGAAGTACTGGATGTTTCTGCTAACA
GAACCGCTGCTGACGGAACAGTTGAATGGCTTATCCCAACAGTAACTGCAGC
TCCAGGCCAGACGGTCACTATGCCCGTAGTAGTCAAGAGTTCAAGTCTTGCA
GTTGCTGGTGCGCAGTTCAAGATCCAGGCGGCGACAGGCGTAAGTTATTCGT
CCAAGACGGACGGTGACGCTTACGGTTCAGGCATTGTGTACAATAATAGTAA
GTATGCTTTTGGACAGGGTGCAGGTAGAGGAATAGTTGCAGCTGATGATTCG
GTTGTGCTTACTCTTGCATATACAGTTCCCGCTGATTGTGCTGAAGGTACAT
ATGATGTCAAGTGGTCTGATGCGTTTGTAAGTGATACAGACGGACAGAATAT
CACAAGTAAGGTTACTCTTACTGATGGCGCTATCATTGTTAAGCTCGAGCAC
CACCACCACCACCACTGA
>CsgA-Scaf6 protein 
MKLLKVAAIAAIVFSGSALAGVVPQYGGGGNHGGGGNNSGPNSELNIYQYGG
GNSALALQTDARNSDLTITQHGGGNGADVGQGSDDSSIDLTQRGFGNSATLD
QWNGKNSEMTVKQFGGGNGAAVDQTASNSSVNVTQVGFGNNATAHQYASGDS
LKVTVGTANGKPGDTVTVPVTFADVAKMKNVGTCNFYLGYDASLLEVVSVDA
 
GPIVKNAAVNFSSSASNGTISFLFLDNTITDELITADGVFANIKFKLKSVTA
KTTTPVTFKDGGAFGDGTMSKIASVTKTNGSVTIDPTKGATPTNTATPTKSA
TATPTRPSVPTNTPTNTPANTPVSGNLKVEFYNSNPSDTTNSINPQFKVTNT
GSSAIDLSKLTLRYYYTVDGQKDQTFWCDHAAIIGSNGSYNGITSNVKGTFV
KMSSSTNNADTYLEISFTGGTLEPGAHVQIQGRFAKNDWSNYTQSNDYSFKS
ASQFVEWDQVTAYLNGVLVWGKEPGGSVVPSTQPVTTPPATTKPPATTIPPS
DDPNAIKIKVDTVNAKPGDTVNIPVRFSGIPSKGIANCDFVYSYDPNVLEII
EIKPGELIVDPNPDKSFDTAVYPDRKIIVFLFAEDSGTGAYAITKDGVFATI
VAKVKSGAPNGLSVIKFVEVGGFANNDLVEQRTQFFDGGVNVGDIGSAGGLS
AVQPNVSLGEVLDVSANRTAADGTVEWLIPTVTAAPGQTVTMPVVVKSSSLA
VAGAQFKIQAATGVSYSSKTDGDAYGSGIVYNNSKYAFGQGAGRGIVAADDS
VVLTLAYTVPADCAEGTYDVKWSDAFVSDTDGQNITSKVTLTDGAIIVKLEH
HHHHH-
>Cel48Ft DNA 
ATGGCTTCAAGTCCTGCAAACAAGGTGTACCAGGATCGTTTTGAATCCATGT
ACAGCAAGATTAAGGATCCTGCAAACGGATACTTCAGTGAACAGGGAATTCC
TTACCACTCAATTGAAACACTGATGGTCGAAGCTCCTGACTACGGACATGTT
ACAACAAGTGAAGCTATGTCCTATTATATGTGGTTGGAAGCAATGCACGGAA
GATTTTCAGGAGATTTTACAGGTTTTGATAAGTCTTGGTCTGTTACCGAACA
GTATTTGATCCCAACAGAAAAGGATCAGCCCAATACAAGTATGAGCAGATAT
GATGCTAACAAACCGGCTACATACGCTCCGGAATTTCAGGACCCAAGCAAGT
ATCCTTCTCCGTTGGATACTAGTCAACCTGTTGGTAGAGATCCAATCAACTC
ACAATTGACTTCAGCATACGGAACAAGCATGCTGTATGGAATGCACTGGATA
CTTGACGTTGATAACTGGTATGGATTTGGAGCAAGAGCTGATGGAACCTCCA
AGCCATCATATATCAATACTTTCCAAAGAGGTGAGCAGGAGTCAACTTGGGA
AACCATACCTCAACCATGTTGGGATGAACATAAATTTGGTGGACAGTACGGA
TTCCTGGATCTCTTTACAAAGGATACAGGTACTCCGGCAAAGCAATTCAAAT
ATACAAATGCTCCAGATGCTGATGCTCGTGCAGTTCAAGCAACTTACTGGGC
TGATCAGTGGGCAAAAGAACAAGGTAAGAGCGTAAGTACTAGCGTAGGTAAG
 
GCAACAAAGATGGGTGATTACCTTAGATATTCATTCTTTGATAAGTATTTCA
GAAAGATCGGACAACCTTCTCAGGCTGGTACCGGATATGATGCAGCACATTA
TCTGCTTTCATGGTACTATGCATGGGGTGGTGGAATTGACTCTACCTGGTCT
TGGATAATCGGTAGCAGTCATAATCATTTCGGTTACCAGAACCCATTTGCAG
CTTGGGTACTTTCAACAGATGCAAACTTCAAGCCTAAGTCATCAAATGGTGC
ATCAGACTGGGCAAAGAGTTTGGACAGACAGCTTGAATTCTATCAGTGGTTG
CAGTCAGCAGAAGGTGCTATTGCCGGTGGAGCTACAAACTCATGGAACGGAC
GTTATGAAGCAGTTCCTTCAGGTACATCAACATTCTATGGAATGGGTTATGT
AGAAAACCCTGTATATGCTGACCCAGGTAGTAACACTTGGTTTGGTATGCAG
GTATGGTCAATGCAGCGTGTAGCTGAATTGTACTATAAGACTGGCGATGCCA
GAGCTAAGAAACTCTTAGACAAATGGGCAAAATGGATTAATGGCGAAATCAA
GTTCAATGCTGACGGAACATTCCAGATTCCTAGCACAATTGATTGGGAAGGA
CAGCCGGATACTTGGAATCCAACACAGGGATACACCGGAAATGCAAACTTGC
ATGTTAAAGTTGTTAACTATGGTACTGACCTAGGTTGTGCTTCTTCACTTGC
AAACACATTGACTTACTATGCTGCTAAATCAGGAGATGAAACTTCAAGGCAG
AATGCACAGAAATTACTTGACGCTATGTGGAATAACTATAGCGATTCAAAGG
GTATATCAACTGTTGAACAGCGTGGTGATTACCATAGATTCCTTGATCAGGA
AGTTTTTGTACCAGCTGGTTGGACTGGAAAAATGCCTAACGGCGACGTAATC
AAATCTGGTGTCAAGTTCATAGACATTCGTTCCAAGTACAAGCAGGATCCTG
AATGGCAGACAATGGTTGCTGCATTACAGGCAGGACAGGTTCCAACTCAGAG
ATTACACCGTTTCTGGGCTCAGAGTGAATTTGCAGTTGCAAATGGAGTTTAT
GCAATACTCTTCCCAGATCAAGGTCCTGATGGTACTCCTTCTACTAAATTAT
ACGGCGACGTCAATGATGACGGAAAAGTTAACTCAACTGACGCTGTAGCATT
GAAGAGATATGTTTTGAGATCAGGTATAAGCATCAACACTGACAATGCCGAT
TTGAATGAAGACGGCAGAGTTAATTCAACTGACTTAGGAATTTTGAAGAGAT
ATATTCTCAAAGAAATAGATACATTGCCGTACAAGAACCTCGAGCACCACCA
CCACCACCACTGA
>Cel48Ft protein 
MASSPANKVYQDRFESMYSKIKDPANGYFSEQGIPYHSIETLMVEAPDYGHV
TTSEAMSYYMWLEAMHGRFSGDFTGFDKSWSVTEQYLIPTEKDQPNTSMSRY
DANKPATYAPEFQDPSKYPSPLDTSQPVGRDPINSQLTSAYGTSMLYGMHWI
LDVDNWYGFGARADGTSKPSYINTFQRGEQESTWETIPQPCWDEHKFGGQYG
FLDLFTKDTGTPAKQFKYTNAPDADARAVQATYWADQWAKEQGKSVSTSVGK
ATKMGDYLRYSFFDKYFRKIGQPSQAGTGYDAAHYLLSWYYAWGGGIDSTWS
WIIGSSHNHFGYQNPFAAWVLSTDANFKPKSSNGASDWAKSLDRQLEFYQWL
QSAEGAIAGGATNSWNGRYEAVPSGTSTFYGMGYVENPVYADPGSNTWFGMQ
VWSMQRVAELYYKTGDARAKKLLDKWAKWINGEIKFNADGTFQIPSTIDWEG
QPDTWNPTQGYTGNANLHVKVVNYGTDLGCASSLANTLTYYAAKSGDETSRQ
NAQKLLDAMWNNYSDSKGISTVEQRGDYHRFLDQEVFVPAGWTGKMPNGDVI
KSGVKFIDIRSKYKQDPEWQTMVAALQAGQVPTQRLHRFWAQSEFAVANGVY
AILFPDQGPDGTPSTKLYGDVNDDGKVNSTDAVALKRYVLRSGISINTDNAD
LNEDGRVNSTDLGILKRYILKEIDTLPYKNLEHHHHHH-
>Cel9Gc DNA 
ATGGGAACATATAACTATGGAGAAGCATTACAGAAATCAATAATGTTCTATG
AATTCCAGCGTTCGGGAGATCTTCCGGCTGATAAACGTGACAACTGGAGA
CGATTCCGGTATGAAAGACGGTTCTGATGTAGGAGTTGATCTTACAGGAGGA
TGGTACGATGCAGGTGACCATGTGAAATTTAATCTACCTATGTCATATACAT
CTGCAATGCTTGCATGGTCCTTATATGAGGATAAGGATGCTTATGATAAGAG
CGGTCAGACAAAATATATAATGGACGGTATAAAATGGGCTAATGATTATTTT
ATTAAATGTAATCCGACACCCGGTGTATATTATTACCAAGTAGGAGACGGCG
GAAAGGACCACTCTTGGTGGGGCCCTGCGGAAGTAATGCAGATGGAAAGACC
GTCTTTTAAGGTTGACGCTTCTAAGCCCGGTTCTGCAGTATGTGCTTCCACT
GCAGCTTCTCTGGCATCTGCAGCAGTAGTCTTTAAATCCAGTGATCCTACTT
ATGCAGAAAAGTGCATAAGCCATGCAAAGAACCTGTTTGATATGGCTGACAA
AGCAAAGAGTGATGCTGGTTATACTGCGGCTTCAGGCTACTACAGCTCAAGC
TCATTTTACGATGATCTCTCATGGGCTGCAGTATGGTTATATCTTGCTACAA
ATGACAGTACATATTTAGACAAAGCAGAATCCTATGTACCGAATTGGGGTAA
 
AGAACAGCAGACAGATATTATCGCCTACAAGTGGGACAGTGCTGGGATGAT
GTTCATTATGGTGCTGAGCTTCTTCTTGCAAAGCTTACAAACAAACAATTGT
ATAAGGATAGTATAGAAATGAACCTTGACTTCTGGACAACTGGTGTTAACGG
AACACGTGTTTCTTACACGCCAAAGGGTTTGGCGTGGCTATTCCAATGGGGT
TCATTAAGACATGCTACAACTCAGGCTTTTTAGCCGGTGTTTATGCAGAGT
GGGAAGGCTGTACGCCATCCAAAGTATCTGTATATAAGGATTTCCTCAAGAG
TCAAATTGATTATGCACTTGGCAGTACCGGAAGAAGTTTTGTTGTCGGATAT
GGAGTAAATCCTCCTCAACATCCTCATCACAGAACTGCTCACGGTTCATGGA
CAGATCAAATGACTTCACCAACATACCACAGGCATACTATTTATGGTGCGTT
GGTAGGAGGACCGGATAATGCAGATGGCTATACTGATGAAATAAACAATTAT
GTCAATAATGAAATAGCCTGCGATTATAATGCCGGATTTACAGGTGCACTTG
CAAAAATGTACAAGCATTCTGGCGGAGATCCGATTCCAAACTTCAAGGCTAT
CGAAAAAATAACCAACGATGAAGTTATTATAAAGGCAGGTTTGAATTCAACT
GGCCCTAACTACACTGAAATCAAGGCTGTTGTTTATAACCAGACAGGATGGC
CTGCAAGAGTTACGGACAAGATATCATTTAAATATTTTATGGACTTGTCTGA
AATTGTAGCAGCAGGAATTGATCCTTTAAGCCTTGTAACAAGTTCAAATTAT
TCTGAAGGTAAGAATACTAAGGTTTCCGGTGTGTTGCCATGGGATGTTTCAA
ATAATGTTTACTATGTAAATGTTGATTTGACAGGAGAAAATATCTACCCAGG
CGGTCAGTCTGCGTGCAGACGAGAAGTTCAGTTCAGAATTGCCGCACCACAG
GGAACAACATATTGGAATCCGAAAAATGATTTCTCATATGATGGATTACCAA
CCACCAGTACTGTAAATACGGTTACCAACATACCTGTTTATGATAACGGCGT
AAAAGTATTTGGTAACGAACCCGCAGGTGGATCGGAAAACCCTGATCCTGAA
ATCTTGTATGGAGACGTAAACAGCGACAAAAATGTAGATGCATTGGACTTTG
CTGCATTGAAGAAATATTTACTTGGAGGCACTTCCAGCATAGATGTTAAGGC
TGCAGATACATACAAGGATGGGAATATTGACGCTATAGATATGGCTACCTTG
AAGAAGTATTTATTGGGAACAATCACCCAATTACCTCAAGGCCTCGAGCACC
ACCACCACCACCACTGA
>Cel9Gc protein 
MGTYNYGEALQKSIMFYEFQRSGDLPADKRDNWRDDSGMKDGSDVGVDLTGG
WYDAGDHVKFNLPMSYTSAMLAWSLYEDKDAYDKSGQTKYIMDGIKWANDYF
IKCNPTPGVYYYQVGDGGKDHSWWGPAEVMQMERPSFKVDASKPGSAVCAST
AASLASAAVVFKSSDPTYAEKCISHAKNLFDMADKAKSDAGYTAASGYYSSS
SFYDDLSWAAVWLYLATNDSTYLDKAESYVPNWGKEQQTDIIAYKWGQCWDD
VHYGAELLLAKLTNKQLYKDSIEMNLDFWTTGVNGTRVSYTPKGLAWLFQWG
SLRHATTQAFLAGVYAEWEGCTPSKVSVYKDFLKSQIDYALGSTGRSFVVGY
GVNPPQHPHHRTAHGSWTDQMTSPTYHRHTIYGALVGGPDNADGYTDEINNY
VNNEIACDYNAGFTGALAKMYKHSGGDPIPNFKAIEKITNDEVIIKAGLNST
GPNYTEIKAVVYNQTGWPARVTDKISFKYFMDLSEIVAAGIDPLSLVTSSNY
SEGKNTKVSGVLPWDVSNNVYYVNVDLTGENIYPGGQSACRREVQFRIAAPQ
GTTYWNPKNDFSYDGLPTTSTVNTVTNIPVYDNGVKVFGNEPAGGSENPDPE
ILYGDVNSDKNVDALDFAALKKYLLGGTSSIDVKAADTYKDGNIDAIDMATL
KKYLLGTITQLPQGLEHHHHHH-
>Cel5Af DNA 
ATGTATGATGCTTCACTTATTCCGAATCTTCAGATTCCACAAAAGAACATTC
CGAATAATGATGGAATGAATTTTGTAAAAGGTTTAAGACTCGGATGGAATCT
GGGTAATACATTTGATGCTTTTAACGGTACAAATATTACTAATGAATTGGAT
TATGAAACATCATGGAGCGGTATCAAAACAACTAAGCAGATGATAGATGCAA
TAAAGCAAAAAGGATTCAATACTGTTCGTATTCCTGTATCCTGGCATCCACA
CGTAAGTGGTTCAGATTACAAAATCAGTGATGTATGGATGAATCGTGTTCAA
GAAGTAGTAAATTATTGTATAGATAATAAAATGTATGTCATTTTAAACACAC
ATCATGACGTTGACAAAGTAAAAGGTTATTTCCCAAGCAGTCAATATATGGC
AAGCTCCAAGAAATATATAACTAGTGTCTGGGCACAGATTGCTGCTAGGTTT
GCAAACTATGATGAGCATCTTATTTTTGAAGGAATGAACGAGCCTCGTCTTG
TAGGACATGCAAATGAGTGGTGGCCTGAGCTGACAAATTCAGATGTAGTTGA
TTCTATTAATTGTATTAATCAACTTAATCAGGATTTTGTTAATACAGTACGT
GCAACAGGTGGAAAAAATGCAAGCAGATATCTTATGTGTCCAGGATATGTTG
CATCTCCTGACGGAGCAACAAACGATTACTTCAGAATGCCAAATGATATTTC
 
TGGTAATAACAACAAAATAATTGTATCTGTACATGCATATTGTCCATGGAAT
TTTGCAGGGTTGGCAATGGCTGATGGAGGTACAAATGCTTGGAATATAAATG
ATTCAAAAGATCAAAGTGAAGTTACTTGGTTTATGGATAATATTTATAATAA
GTATACAAGCAGGGGTATTCCTGTAATAATCGGTGAATGTGGAGCAGTAGAT
AAGAACAATCTGAAGACAAGAGTAGAATATATGTCCTATTATGTTGCACAAG
CTAAAGCACGTGGTATATTATGCATATTGTGGGATAACAATAATTTCTCAGG
TACTGGTGAATTATTTGGTTTCTTCGATAGAAGAAGCTGTCAGTTCAAGTTC
CCTGAAATTATAGATGGAATGGTGAAATATGCTTTCGAAGCCAAGACAGATC
CTGACCCCGGCACAAAGCTCGTTCCTACATGGGGCGATACAAACTGCGACGG
CGTTGTAAATGTTGCTGACGTAGTAGTTCTTAACAGATTCCTCAACGATCCT
ACATATTCTAACATTACTGATCAGGGTAAGGTTAACGCAGACGTTGTTGATC
CTCAGGATAAGTCCGGCGCAGCAGTTGATCCTGCAGGCGTAAAGCTCACAGT
AGCTGACTCTGAGGCAATCCTCAAGGCTATCGTTGAACTCATCACACTTCCT
CAACTCGAGCACCACCACCACCACCACTGA
>Cel5Af protein

MYDASLIPNLQIPQKNIPNNDGMNFVKGLRLGWNLGNTFDAFNGTNITNELD
YETSWSGIKTTKQMIDAIKQKGFNTVRIPVSWHPHVSGSDYKISDVWMNRVQ
EVVNYCIDNKMYVILNTHHDVDKVKGYFPSSQYMASSKKYITSVWAQIAARF
ANYDEHLIFEGMNEPRLVGHANEWWPELTNSDVVDSINCINQLNQDFVNTVR
ATGGKNASRYLMCPGYVASPDGATNDYFRMPNDISGNNNKIIVSVHAYCPWN
FAGLAMADGGTNAWNINDSKDQSEVTWFMDNIYNKYTSRGIPVIIGECGAVD
KNNLKTRVEYMSYYVAQAKARGILCILWDNNNFSGTGELFGFFDRRSCQFKF
PEIIDGMVKYAFEAKTDPDPGTKLVPTWGDTNCDGVVNVADVVVLNRFLNDP
TYSNITDQGKVNADVVDPQDKSGAAVDPAGVKLTVADSEAILKAIVELITLP
QLEHHHHHH-

## Claims

1. A recombinant modified *Gammaproteobacteriaceae* that displays on its cell surface a modified Curli system comprising:
i) a CsgA protein or a CsgA homologous protein linked to the surface of said *Gammaproteobacteriaceae;* and
ii) a scaffoldin comprising at least one cellulosomal component, said scaffoldin being fused to the C-terminus of the CsgA protein.

2. The recombinant modified *Gammaproteobacteriaceae* according to claim 1, wherein the modified Curli system is endogenous or exogenous, or a combination of endogenous and exogenous components.

3. The recombinant modified *Gammaproteobacteriaceae* according to any one of claims 1 or 2, wherein the *Gammaproteobacteriaceae* is selected from the group consisting of *Escherichia, Enterobacter, Salmonella, Sodalis, Pectobacterium, Photorhabdus, Serratia, Yersinia, Buchnera, Vibrio,* Shewanella and *Pseudomonas,* preferably Escherichia, more preferably Escherichia coli.

4. The recombinant modified *Gammaproteobacteriaceae* according to any one of claims 1 to 3, wherein the at least one cellulosomal component is selected from the group consisting of a carbohydrate binding module, a cohesin domain and a dockerin domain, said module or domains originating from any cellulosome-producing microorganism, and is preferably a carbohydrate binding module.

5. The recombinant modified *Gammaproteobacteriaceae* according to any one of claims 3 or 4, wherein the at least one cellulosomal component comprises a cohesin domain, said cohesin domain being selected from the group consisting of a cohesin domain from *C. cellulolyticum,* a cohesin domain from *C. thermocellum,* a cohesin domain from *R*. *flavefaciens,* and any cohesin domain from any cellulosome-producing microorganism.

6. The recombinant modified *Gammaproteobacteriaceae* according to any one of claims 3, 4 or 5, wherein the at least one cellulosomal component comprises a dockerin domain, said dockerin domain being selected from the group consisting of a dockerin domains from *C. cellulolyticum,* a dockerin domain from *C. thermocellum,* a dockerin domain from *R. flavefaciens,* and any dockerin domain from any cellulosome-producing microorganism.

7. The recombinant modified *Gammaproteobacteriaceae* according to any one of claims 1 to 6, wherein the cellulosomal component comprises any combination of the module or domains of claim 5.

8. The recombinant modified *Gammaproteobacteriaceae* according to claim 7, wherein the cellulosomal component comprises at least one cohesin domains and a carbohydrate binding module.

9. The recombinant modified *Gammaproteobacteriaceae* according to any one of claims 3 to 8, wherein the cohesin domain is tightly bound to at least one protein harboring a dockerin fused to at least one cellulolytic enzyme, and wherein the dockerin is anchored to said cohesin domain.

10. The recombinant modified *Gammaproteobacteriaceae* according to claim 9, wherein the protein harboring the dockerin further comprises a carbohydrate binding module.

11. The recombinant modified *Gammaproteobacteriaceae* according to any one of claims 9 and 10, wherein the dockerin is selected from the group consisting of dockerin domain from *C. cellulolyticum,* dockerin domain from *C. thermocellum,* dockerin domain from *R. flavefaciens,* and any dockerin from any cellulosome-producing microorganism.

12. Use of the recombinant modified *Gammaproteobacteriaceae* according to any one of claims 1 to 11, for degrading lignocellulosic biomass.

13. Use of the recombinant modified *Gammaproteobacteriaceae* according to any one of claims 1 to 11, as a biocatalyst.

14. Use of the recombinant modified *Gammaproteobacteriaceae* according to any one of claims 1 to 11, as a cellular vector for vaccination.

15. Use of the recombinant modified *Gammaproteobacteriaceae* according to any one of claims 1 to 11, for bioremediation of toxic compounds or metals.


SEQUENCE LISTING

<110> Centre National de la Recherche Scientifique (CNRS)
Université d'Aix Marseille (AMU)
Université d'Aix Marseille (AMU)

<120> RECOMBINANT MODIFIED MICROORGANISMS DISPLAYING MEMBRANE
FUNCTIONALIZATION FOR VARIOUS BIOTECHNOLOGICAL APPLICATIONS

<130> 11161EP

<160> 18

<170> PatentIn version 3.5

<210> 1
<211> 31
<212> DNA
<213> Unknown

<220>
<223> Primer

<400> 1
gttggagata taggatcagc cggtggttta t 31

<210> 2
<211> 31
<212> DNA
<213> Unknown

<220>
<223> Primer

<400> 2
ataaaccacc ggctgatcct atatctccaa c 31

<210> 3
<211> 33
<212> DNA
<213> Unknown

<220>
<223> Primer

<400> 3
ggggggctag cggcgattct cttaaagtta cag 33

<210> 4
<211> 34
<212> DNA
<213> Unknown

<220>
<223> Primer

<400> 4
gggggctcga gcttaacaat gatagcgcca tcag 34

<210>  5
<211>  34
<212>  DNA
<213>  Unknown

<220>
<223>  Primer

<400>  5
gggggggatc catgaaactt ttaaaagtag cagc                                    34


<210>  6
<211>  31
<212>  DNA
<213>  Unknown

<220>
<223>  Primer

<400>  6
ggggggctag cgtactgatg agcggtcgcg t                                        31


<210>  7
<211>  18
<212>  DNA
<213>  Unknown

<220>
<223>  Overlapping oligomer

<400>  7
ctagctgatg accgcggc                                                      18


<210>  8
<211>  18
<212>  DNA
<213>  Unknown

<220>
<223>  Overlapping oliomer

<400>  8
tcgagccgcg gtcatcag                                                      18


<210>  9
<211>  462
<212>  DNA
<213>  Escherichia coli

<400>  9
atgaaacttt taaaagtagc agcaattgca gcaatcgtat tctccggtag cgctctggca      60

ggtgttgttc ctcagtacgg cggcggcggt aaccacggtg gtggcggtaa taatagcggc     120

ccaaattctg agctgaacat ttaccagtac ggtggcggta actctgcact tgctctgcaa     180

actgatgccc gtaactctga cttgactatt acccagcatg gcggcggtaa tggtgcagat     240

gttggtcagg gctcagatga cagctcaatc gatctgaccc aacgtggctt cggtaacagc     300

gctactcttg atcagtggaa cggcaaaaat tctgaaatga cggttaaaca gttcggtggt     360

ggcaacggtg ctgcagttga ccagactgca tctaactcct ccgtcaacgt gactcaggtt     420

ggctttggta acaacgcgac cgctcatcag tacgctagct ga                        462

<210>  10
<211>  153
<212>  PRT
<213>  Escherichia coli

<400>  10

Met Lys Leu Leu Lys Val Ala Ala Ile Ala Ala Ile Val Phe Ser Gly
1               5                   10                  15

Ser Ala Leu Ala Gly Val Val Pro Gln Tyr Gly Gly Gly Gly Asn His
            20                  25                  30

Gly Gly Gly Gly Asn Asn Ser Gly Pro Asn Ser Glu Leu Asn Ile Tyr
        35                  40                  45

Gln Tyr Gly Gly Gly Asn Ser Ala Leu Ala Leu Gln Thr Asp Ala Arg
    50                  55                  60

Asn Ser Asp Leu Thr Ile Thr Gln His Gly Gly Gly Asn Gly Ala Asp
65                  70                  75                  80

Val Gly Gln Gly Ser Asp Asp Ser Ser Ile Asp Leu Thr Gln Arg Gly
                85                  90                  95

Phe Gly Asn Ser Ala Thr Leu Asp Gln Trp Asn Gly Lys Asn Ser Glu
            100                 105                 110

Met Thr Val Lys Gln Phe Gly Gly Gly Asn Gly Ala Ala Val Asp Gln
        115                 120                 125

Thr Ala Ser Asn Ser Ser Val Asn Val Thr Gln Val Gly Phe Gly Asn
    130                 135                 140

Asn Ala Thr Ala His Gln Tyr Ala Ser
145                 150

<210>  11
<211>  2514
<212>  DNA
<213>  Escherichia coli

<400>  11
atgaaacttt taaaagtagc agcaattgca gcaatcgtat tctccggtag cgctctggca      60

ggtgttgttc ctcagtacgg cggcggcggt aaccacggtg gtggcggtaa taatagcggc 120

ccaaattctg agctgaacat ttaccagtac ggtggcggta actctgcact tgctctgcaa 180

actgatgccc gtaactctga cttgactatt acccagcatg gcggcggtaa tggtgcagat 240

gttggtcagg gctcagatga cagctcaatc gatctgaccc aacgtggctt cggtaacagc 300

gctactcttg atcagtggaa cggcaaaaat tctgaaatga cggttaaaca gttcggtggt 360

ggcaacggtg ctgcagttga ccagactgca tctaactcct ccgtcaacgt gactcaggtt 420

ggctttggta acaacgcgac cgctcatcag tacgctagcg gcgattctct taaagttaca 480

gtaggaacag ctaatggtaa gcctggcgat acagtaacag ttcctgttac atttgctgat 540

gtagcaaaga tgaaaaacgt aggaacatgt aatttctatc ttggatatga tgcaagcctg 600

ttagaggtag tatcagtaga tgcaggtcca atagttaaga atgcagcagt taacttctca 660

agcagtgcaa gcaacggaac aatcagcttc ctgttcttgg ataacacaat tacagacgaa 720

ttgataactg cagacggtgt gtttgcaaat attaagttca aattaaagag tgtaacggct 780

aaaactacaa caccagtaac atttaaagat ggtggagctt ttggtgacgg aactatgtca 840

aagatagctt cagttactaa gacaaacggt agtgtaacga tcgatccgac caagggagca 900

acaccaacaa atacagctac gccgacaaaa tcagctacgg ctacgcccac caggccatcg 960

gtaccgacaa acacaccgac aaacacaccg gcaaatacac cggtatcagg caatttgaag 1020

gttgaattct acaacagcaa tccttcagat actactaact caatcaatcc tcagttcaag 1080

gttactaata ccggaagcag tgcaattgat ttgtccaaac tcacattgag atattattat 1140

acagtagacg gacagaaaga tcagaccttc tggtgtgacc atgctgcaat aatcggcagt 1200

aacggcagct acaacggaat tacttcaaat gtaaaaggaa catttgtaaa aatgagttcc 1260

tcaacaaata acgcagacac ctaccttgaa ataagcttta caggcggaac tcttgaaccg 1320

ggtgcacatg ttcagataca aggtagattt gcaaagaatg actggagtaa ctatacacag 1380

tcaaatgact actcattcaa gtctgcttca cagtttgttg aatgggatca ggtaacagca 1440

tacttgaacg gtgttcttgt atggggtaaa gaacccggtg gcagtgtagt accatcaaca 1500

cagcctgtaa caacaccacc tgcaacaaca aaaccacctg caacaacaat accgccgtca 1560

gatgatccga atgcaataaa gattaaggtg gacacagtaa atgcaaaacc gggagacaca 1620

gtaaatatac ctgtaagatt cagtggtata ccatccaagg gaatagcaaa ctgtgacttt 1680

gtatacagct atgacccgaa tgtacttgag ataatagaga taaaaccggg agaattgata 1740

gttgacccga atcctgacaa gagctttgat actgcagtat atcctgacag aaagataata 1800

gtattcctgt ttgcagaaga cagcggaaca ggagcgtatg caataactaa agacggagta 1860

tttgctacga tagtagcgaa agtaaaatcc ggagcaccta acggactcag tgtaatcaaa 1920

tttgtagaag taggcggatt tgcgaacaat gaccttgtag aacagaggac acagttcttt 1980

gacggtggag taaatgttgg agatatagga tcagccggtg gtttatccgc tgtgcagcct     2040

aatgttagtt taggcgaagt actggatgtt tctgctaaca gaaccgctgc tgacggaaca     2100

gttgaatggc ttatcccaac agtaactgca gctccaggcc agacggtcac tatgcccgta     2160

gtagtcaaga gttcaagtct tgcagttgct ggtgcgcagt tcaagatcca ggcggcgaca     2220

ggcgtaagtt attcgtccaa gacggacggt gacgcttacg gttcaggcat tgtgtacaat     2280

aatagtaagt atgcttttgg acagggtgca ggtagaggaa tagttgcagc tgatgattcg     2340

gttgtgctta ctcttgcata tacagttccc gctgattgtg ctgaaggtac atatgatgtc     2400

aagtggtctg atgcgtttgt aagtgataca gacggacaga atatcacaag taaggttact     2460

cttactgatg gcgctatcat tgttaagctc gagcaccacc accaccacca ctga           2514

<210>  12
<211>  837
<212>  PRT
<213>  Escherichia coli

<400>  12

Met Lys Leu Leu Lys Val Ala Ala Ile Ala Ala Ile Val Phe Ser Gly
1               5                   10                  15

Ser Ala Leu Ala Gly Val Val Pro Gln Tyr Gly Gly Gly Gly Asn His
            20                  25                  30

Gly Gly Gly Gly Asn Asn Ser Gly Pro Asn Ser Glu Leu Asn Ile Tyr
        35                  40                  45

Gln Tyr Gly Gly Gly Asn Ser Ala Leu Ala Leu Gln Thr Asp Ala Arg
    50                  55                  60

Asn Ser Asp Leu Thr Ile Thr Gln His Gly Gly Gly Asn Gly Ala Asp
65                  70                  75                  80

Val Gly Gln Gly Ser Asp Asp Ser Ser Ile Asp Leu Thr Gln Arg Gly
                85                  90                  95

Phe Gly Asn Ser Ala Thr Leu Asp Gln Trp Asn Gly Lys Asn Ser Glu
            100                 105                 110

Met Thr Val Lys Gln Phe Gly Gly Gly Asn Gly Ala Ala Val Asp Gln
        115                 120                 125

Thr Ala Ser Asn Ser Ser Val Asn Val Thr Gln Val Gly Phe Gly Asn
    130                 135                 140

Asn Ala Thr Ala His Gln Tyr Ala Ser Gly Asp Ser Leu Lys Val Thr
145                 150                 155                 160

Val Gly Thr Ala Asn Gly Lys Pro Gly Asp Thr Val Thr Val Pro Val
                165                 170                 175

Thr Phe Ala Asp Val Ala Lys Met Lys Asn Val Gly Thr Cys Asn Phe
            180                 185                 190

Tyr Leu Gly Tyr Asp Ala Ser Leu Leu Glu Val Val Ser Val Asp Ala
        195                 200                 205

Gly Pro Ile Val Lys Asn Ala Ala Val Asn Phe Ser Ser Ser Ala Ser
    210                 215                 220

Asn Gly Thr Ile Ser Phe Leu Phe Leu Asp Asn Thr Ile Thr Asp Glu
225                 230                 235                 240

Leu Ile Thr Ala Asp Gly Val Phe Ala Asn Ile Lys Phe Lys Leu Lys
                245                 250                 255

Ser Val Thr Ala Lys Thr Thr Thr Pro Val Thr Phe Lys Asp Gly Gly
            260                 265                 270

Ala Phe Gly Asp Gly Thr Met Ser Lys Ile Ala Ser Val Thr Lys Thr
        275                 280                 285

Asn Gly Ser Val Thr Ile Asp Pro Thr Lys Gly Ala Thr Pro Thr Asn
    290                 295                 300

Thr Ala Thr Pro Thr Lys Ser Ala Thr Ala Thr Pro Thr Arg Pro Ser
305                 310                 315                 320

Val Pro Thr Asn Thr Pro Thr Asn Thr Pro Ala Asn Thr Pro Val Ser
                325                 330                 335

Gly Asn Leu Lys Val Glu Phe Tyr Asn Ser Asn Pro Ser Asp Thr Thr
            340                 345                 350

Asn Ser Ile Asn Pro Gln Phe Lys Val Thr Asn Thr Gly Ser Ser Ala
        355                 360                 365

Ile Asp Leu Ser Lys Leu Thr Leu Arg Tyr Tyr Tyr Thr Val Asp Gly
    370                 375                 380

Gln Lys Asp Gln Thr Phe Trp Cys Asp His Ala Ala Ile Ile Gly Ser
385                 390                 395                 400

Asn Gly Ser Tyr Asn Gly Ile Thr Ser Asn Val Lys Gly Thr Phe Val
                405                 410                 415

Lys Met Ser Ser Ser Thr Asn Asn Ala Asp Thr Tyr Leu Glu Ile Ser
            420                 425                 430

Phe Thr Gly Gly Thr Leu Glu Pro Gly Ala His Val Gln Ile Gln Gly
        435                 440                 445

Arg Phe Ala Lys Asn Asp Trp Ser Asn Tyr Thr Gln Ser Asn Asp Tyr
    450                 455                 460

Ser Phe Lys Ser Ala Ser Gln Phe Val Glu Trp Asp Gln Val Thr Ala
465                 470                 475                 480

Tyr Leu Asn Gly Val Leu Val Trp Gly Lys Glu Pro Gly Gly Ser Val
                485                 490                 495

Val Pro Ser Thr Gln Pro Val Thr Thr Pro Pro Ala Thr Thr Lys Pro
            500                 505                 510

Pro Ala Thr Thr Ile Pro Pro Ser Asp Asp Pro Asn Ala Ile Lys Ile
        515                 520                 525

Lys Val Asp Thr Val Asn Ala Lys Pro Gly Asp Thr Val Asn Ile Pro
    530                 535                 540

Val Arg Phe Ser Gly Ile Pro Ser Lys Gly Ile Ala Asn Cys Asp Phe
545                 550                 555                 560

Val Tyr Ser Tyr Asp Pro Asn Val Leu Glu Ile Ile Glu Ile Lys Pro
                565                 570                 575

Gly Glu Leu Ile Val Asp Pro Asn Pro Asp Lys Ser Phe Asp Thr Ala
            580                 585                 590

Val Tyr Pro Asp Arg Lys Ile Ile Val Phe Leu Phe Ala Glu Asp Ser
        595                 600                 605

Gly Thr Gly Ala Tyr Ala Ile Thr Lys Asp Gly Val Phe Ala Thr Ile
    610                 615                 620

Val Ala Lys Val Lys Ser Gly Ala Pro Asn Gly Leu Ser Val Ile Lys
625                 630                 635                 640

Phe Val Glu Val Gly Gly Phe Ala Asn Asn Asp Leu Val Glu Gln Arg
                645                 650                 655

Thr Gln Phe Phe Asp Gly Gly Val Asn Val Gly Asp Ile Gly Ser Ala
            660                 665                 670

Gly Gly Leu Ser Ala Val Gln Pro Asn Val Ser Leu Gly Glu Val Leu
        675                 680                 685

Asp Val Ser Ala Asn Arg Thr Ala Ala Asp Gly Thr Val Glu Trp Leu
    690                 695                 700

Ile Pro Thr Val Thr Ala Ala Pro Gly Gln Thr Val Thr Met Pro Val
705                 710                 715                 720

Val Val Lys Ser Ser Ser Leu Ala Val Ala Gly Ala Gln Phe Lys Ile
                725                 730                 735

Gln Ala Ala Thr Gly Val Ser Tyr Ser Ser Lys Thr Asp Gly Asp Ala
            740                 745                 750

Tyr Gly Ser Gly Ile Val Tyr Asn Asn Ser Lys Tyr Ala Phe Gly Gln
        755                 760                 765

Gly Ala Gly Arg Gly Ile Val Ala Ala Asp Asp Ser Val Val Leu Thr
    770                 775                 780

Leu Ala Tyr Thr Val Pro Ala Asp Cys Ala Glu Gly Thr Tyr Asp Val
785                 790                 795                 800

Lys Trp Ser Asp Ala Phe Val Ser Asp Thr Asp Gly Gln Asn Ile Thr
                805                 810                 815

Ser Lys Val Thr Leu Thr Asp Gly Ala Ile Ile Val Lys Leu Glu His
            820                 825                 830

His His His His His
        835

<210>  13
<211>  2145
<212>  DNA
<213>  Escherichia coli

<400>  13
atggcttcaa gtcctgcaaa caaggtgtac caggatcgtt ttgaatccat gtacagcaag      60

attaaggatc ctgcaaacgg atacttcagt gaacagggaa ttccttacca ctcaattgaa     120

acactgatgg tcgaagctcc tgactacgga catgttacaa caagtgaagc tatgtcctat     180

tatatgtggt tggaagcaat gcacggaaga ttttcaggag attttacagg ttttgataag     240

tcttggtctg ttaccgaaca gtatttgatc ccaacagaaa aggatcagcc caatacaagt 300

atgagcagat atgatgctaa caaaccggct acatacgctc cggaatttca ggacccaagc 360

aagtatcctt ctccgttgga tactagtcaa cctgttggta gagatccaat caactcacaa 420

ttgacttcag catacggaac aagcatgctg tatggaatgc actggatact tgacgttgat 480

aactggtatg gatttggagc aagagctgat ggaacctcca agccatcata tatcaatact 540

ttccaaagag gtgagcagga gtcaacttgg gaaaccatac ctcaaccatg ttgggatgaa 600

cataaatttg gtggacagta cggattcctg gatctcttta caaaggatac aggtactccg 660

gcaaagcaat tcaaatatac aaatgctcca gatgctgatg ctcgtgcagt tcaagcaact 720

tactgggctg atcagtgggc aaaagaacaa ggtaagagcg taagtactag cgtaggtaag 780

gcaacaaaga tgggtgatta ccttagatat tcattctttg ataagtattt cagaaagatc 840

ggacaacctt ctcaggctgg taccggatat gatgcagcac attatctgct ttcatggtac 900

tatgcatggg gtggtggaat tgactctacc tggtcttgga taatcggtag cagtcataat 960

catttcggtt accagaaccc atttgcagct tgggtacttt caacagatgc aaacttcaag 1020

cctaagtcat caaatggtgc atcagactgg gcaaagagtt tggacagaca gcttgaattc 1080

tatcagtggt tgcagtcagc agaaggtgct attgccggtg gagctacaaa ctcatggaac 1140

ggacgttatg aagcagttcc ttcaggtaca tcaacattct atggaatggg ttatgtagaa 1200

aaccctgtat atgctgaccc aggtagtaac acttggtttg gtatgcaggt atggtcaatg 1260

cagcgtgtag ctgaattgta ctataagact ggcgatgcca gagctaagaa actcttagac 1320

aaatgggcaa aatggattaa tggcgaaatc aagttcaatg ctgacggaac attccagatt 1380

cctagcacaa ttgattggga aggacagccg gatacttgga atccaacaca gggatacacc 1440

ggaaatgcaa acttgcatgt taaagttgtt aactatggta ctgacctagg ttgtgcttct 1500

tcacttgcaa acacattgac ttactatgct gctaaatcag gagatgaaac ttcaaggcag 1560

aatgcacaga aattacttga cgctatgtgg aataactata gcgattcaaa gggtatatca 1620

actgttgaac agcgtggtga ttaccataga ttccttgatc aggaagtttt tgtaccagct 1680

ggttggactg gaaaaatgcc taacggcgac gtaatcaaat ctggtgtcaa gttcatagac 1740

attcgttcca agtacaagca ggatcctgaa tggcagacaa tggttgctgc attacaggca 1800

ggacaggttc caactcagag attacaccgt ttctgggctc agagtgaatt tgcagttgca 1860

aatggagttt atgcaatact cttcccagat caaggtcctg atggtactcc ttctactaaa 1920

ttatacggcg acgtcaatga tgacggaaaa gttaactcaa ctgacgctgt agcattgaag 1980

agatatgttt tgagatcagg tataagcatc aacactgaca atgccgattt gaatgaagac 2040

ggcagagtta attcaactga cttaggaatt ttgaagagat atattctcaa agaaatagat 2100

acattgccgt acaagaacct cgagcaccac caccaccacc actga                    2145

<210>  14
<211>  714
<212>  PRT
<213>  Escherichia coli

<400>  14

Met Ala Ser Ser Pro Ala Asn Lys Val Tyr Gln Asp Arg Phe Glu Ser
1               5                   10                  15

Met Tyr Ser Lys Ile Lys Asp Pro Ala Asn Gly Tyr Phe Ser Glu Gln
            20                  25                  30

Gly Ile Pro Tyr His Ser Ile Glu Thr Leu Met Val Glu Ala Pro Asp
        35                  40                  45

Tyr Gly His Val Thr Thr Ser Glu Ala Met Ser Tyr Tyr Met Trp Leu
    50                  55                  60

Glu Ala Met His Gly Arg Phe Ser Gly Asp Phe Thr Gly Phe Asp Lys
65                  70                  75                  80

Ser Trp Ser Val Thr Glu Gln Tyr Leu Ile Pro Thr Glu Lys Asp Gln
                85                  90                  95

Pro Asn Thr Ser Met Ser Arg Tyr Asp Ala Asn Lys Pro Ala Thr Tyr
            100                 105                 110

Ala Pro Glu Phe Gln Asp Pro Ser Lys Tyr Pro Ser Pro Leu Asp Thr
        115                 120                 125

Ser Gln Pro Val Gly Arg Asp Pro Ile Asn Ser Gln Leu Thr Ser Ala
    130                 135                 140

Tyr Gly Thr Ser Met Leu Tyr Gly Met His Trp Ile Leu Asp Val Asp
145                 150                 155                 160

Asn Trp Tyr Gly Phe Gly Ala Arg Ala Asp Gly Thr Ser Lys Pro Ser
                165                 170                 175

Tyr Ile Asn Thr Phe Gln Arg Gly Glu Gln Glu Ser Thr Trp Glu Thr
            180                 185                 190

Ile Pro Gln Pro Cys Trp Asp Glu His Lys Phe Gly Gly Gln Tyr Gly
        195                 200                 205

Phe Leu Asp Leu Phe Thr Lys Asp Thr Gly Thr Pro Ala Lys Gln Phe

210                 215                 220

Lys Tyr Thr Asn Ala Pro Asp Ala Asp Ala Arg Ala Val Gln Ala Thr
225                 230                 235                 240

Tyr Trp Ala Asp Gln Trp Ala Lys Glu Gln Gly Lys Ser Val Ser Thr
                245                 250                 255

Ser Val Gly Lys Ala Thr Lys Met Gly Asp Tyr Leu Arg Tyr Ser Phe
            260                 265                 270

Phe Asp Lys Tyr Phe Arg Lys Ile Gly Gln Pro Ser Gln Ala Gly Thr
        275                 280                 285

Gly Tyr Asp Ala Ala His Tyr Leu Leu Ser Trp Tyr Tyr Ala Trp Gly
    290                 295                 300

Gly Gly Ile Asp Ser Thr Trp Ser Trp Ile Ile Gly Ser Ser His Asn
305                 310                 315                 320

His Phe Gly Tyr Gln Asn Pro Phe Ala Ala Trp Val Leu Ser Thr Asp
                325                 330                 335

Ala Asn Phe Lys Pro Lys Ser Ser Asn Gly Ala Ser Asp Trp Ala Lys
            340                 345                 350

Ser Leu Asp Arg Gln Leu Glu Phe Tyr Gln Trp Leu Gln Ser Ala Glu
        355                 360                 365

Gly Ala Ile Ala Gly Gly Ala Thr Asn Ser Trp Asn Gly Arg Tyr Glu
    370                 375                 380

Ala Val Pro Ser Gly Thr Ser Thr Phe Tyr Gly Met Gly Tyr Val Glu
385                 390                 395                 400

Asn Pro Val Tyr Ala Asp Pro Gly Ser Asn Thr Trp Phe Gly Met Gln
                405                 410                 415

Val Trp Ser Met Gln Arg Val Ala Glu Leu Tyr Tyr Lys Thr Gly Asp
            420                 425                 430

Ala Arg Ala Lys Lys Leu Leu Asp Lys Trp Ala Lys Trp Ile Asn Gly
        435                 440                 445

Glu Ile Lys Phe Asn Ala Asp Gly Thr Phe Gln Ile Pro Ser Thr Ile
    450                 455                 460

Asp Trp Glu Gly Gln Pro Asp Thr Trp Asn Pro Thr Gln Gly Tyr Thr
465                 470                 475                 480

Gly Asn Ala Asn Leu His Val Lys Val Val Asn Tyr Gly Thr Asp Leu
                485                 490                 495

Gly Cys Ala Ser Ser Leu Ala Asn Thr Leu Thr Tyr Tyr Ala Ala Lys
            500                 505                 510

Ser Gly Asp Glu Thr Ser Arg Gln Asn Ala Gln Lys Leu Leu Asp Ala
        515                 520                 525

Met Trp Asn Asn Tyr Ser Asp Ser Lys Gly Ile Ser Thr Val Glu Gln
    530                 535                 540

Arg Gly Asp Tyr His Arg Phe Leu Asp Gln Glu Val Phe Val Pro Ala
545                 550                 555                 560

Gly Trp Thr Gly Lys Met Pro Asn Gly Asp Val Ile Lys Ser Gly Val
                565                 570                 575

Lys Phe Ile Asp Ile Arg Ser Lys Tyr Lys Gln Asp Pro Glu Trp Gln
            580                 585                 590

Thr Met Val Ala Ala Leu Gln Ala Gly Gln Val Pro Thr Gln Arg Leu
        595                 600                 605

His Arg Phe Trp Ala Gln Ser Glu Phe Ala Val Ala Asn Gly Val Tyr
    610                 615                 620

Ala Ile Leu Phe Pro Asp Gln Gly Pro Asp Gly Thr Pro Ser Thr Lys
625                 630                 635                 640

Leu Tyr Gly Asp Val Asn Asp Asp Gly Lys Val Asn Ser Thr Asp Ala
                645                 650                 655

Val Ala Leu Lys Arg Tyr Val Leu Arg Ser Gly Ile Ser Ile Asn Thr
            660                 665                 670

Asp Asn Ala Asp Leu Asn Glu Asp Gly Arg Val Asn Ser Thr Asp Leu
        675                 680                 685

Gly Ile Leu Lys Arg Tyr Ile Leu Lys Glu Ile Asp Thr Leu Pro Tyr
    690                 695                 700

Lys Asn Leu Glu His His His His His His
705                 710

<210>  15
<211>  2097
<212>  DNA
<213>  Escherichia coli

<400>  15
atgggaacat ataactatgg agaagcatta cagaaatcaa taatgttcta tgaattccag      60

cgttcgggag atcttccggc tgataaacgt gacaactgga gagacgattc cggtatgaaa     120

gacggttctg atgtaggagt tgatcttaca ggaggatggt acgatgcagg tgaccatgtg     180

aaatttaatc tacctatgtc atatacatct gcaatgcttg catggtcctt atatgaggat     240

aaggatgctt atgataagag cggtcagaca aaatatataa tggacggtat aaaatgggct     300

aatgattatt ttattaaatg taatccgaca cccggtgtat attattacca agtaggagac     360

ggcggaaagg accactcttg gtggggccct gcggaagtaa tgcagatgga aagaccgtct     420

tttaaggttg acgcttctaa gcccggttct gcagtatgtg cttccactgc agcttctctg     480

gcatctgcag cagtagtctt taaatccagt gatcctactt atgcagaaaa gtgcataagc     540

catgcaaaga acctgtttga tatggctgac aaagcaaaga gtgatgctgg ttatactgcg     600

gcttcaggct actacagctc aagctcattt tacgatgatc tctcatgggc tgcagtatgg     660

ttatatcttg ctacaaatga cagtacatat ttagacaaag cagaatccta tgtaccgaat     720

tggggtaaag aacagcagac agatattatc gcctacaagt ggggacagtg ctgggatgat     780

gttcattatg gtgctgagct tcttcttgca aagcttacaa acaaacaatt gtataaggat     840

agtatagaaa tgaaccttga cttctggaca actggtgtta acggaacacg tgtttcttac     900

acgccaaagg gtttggcgtg gctattccaa tggggttcat taagacatgc tacaactcag     960

gcttttttag ccggtgttta tgcagagtgg gaaggctgta cgccatccaa agtatctgta    1020

tataaggatt tcctcaagag tcaaattgat tatgcacttg gcagtaccgg aagaagtttt    1080

gttgtcggat atggagtaaa tcctcctcaa catcctcatc acagaactgc tcacggttca    1140

tggacagatc aaatgacttc accaacatac cacaggcata ctatttatgg tgcgttggta    1200

ggaggaccgg ataatgcaga tggctatact gatgaaataa acaattatgt caataatgaa    1260

atagcctgcg attataatgc cggatttaca ggtgcacttg caaaaatgta caagcattct    1320

ggcggagatc cgattccaaa cttcaaggct atcgaaaaaa taaccaacga tgaagttatt    1380

ataaaggcag gtttgaattc aactggccct aactacactg aaatcaaggc tgttgtttat    1440

aaccagacag gatggcctgc aagagttacg gacaagatat catttaaata ttttatggac    1500

ttgtctgaaa ttgtagcagc aggaattgat cctttaagcc ttgtaacaag ttcaaattat    1560

tctgaaggta agaatactaa ggtttccggt gtgttgccat gggatgtttc aaataatgtt    1620

tactatgtaa atgttgattt gacaggagaa aatatctacc caggcggtca gtctgcgtgc    1680

agacgagaag ttcagttcag aattgccgca ccacagggaa caacatattg gaatccgaaa 1740

aatgatttct catatgatgg attaccaacc accagtactg taaatacggt taccaacata 1800

cctgtttatg ataacggcgt aaaagtattt ggtaacgaac ccgcaggtgg atcggaaaac 1860

cctgatcctg aaatcttgta tggagacgta aacagcgaca aaaatgtaga tgcattggac 1920

tttgctgcat tgaagaaata tttacttgga ggcacttcca gcatagatgt taaggctgca 1980

gatacataca aggatgggaa tattgacgct atagatatgg ctaccttgaa gaagtattta 2040

ttgggaacaa tcacccaatt acctcaaggc ctcgagcacc accaccacca ccactga 2097

<210> 16
<211> 698
<212> PRT
<213> Escherichia coli

<400> 16

Met Gly Thr Tyr Asn Tyr Gly Glu Ala Leu Gln Lys Ser Ile Met Phe
1               5                   10                  15

Tyr Glu Phe Gln Arg Ser Gly Asp Leu Pro Ala Asp Lys Arg Asp Asn
            20                  25                  30

Trp Arg Asp Asp Ser Gly Met Lys Asp Gly Ser Asp Val Gly Val Asp
        35                  40                  45

Leu Thr Gly Gly Trp Tyr Asp Ala Gly Asp His Val Lys Phe Asn Leu
    50                  55                  60

Pro Met Ser Tyr Thr Ser Ala Met Leu Ala Trp Ser Leu Tyr Glu Asp
65                  70                  75                  80

Lys Asp Ala Tyr Asp Lys Ser Gly Gln Thr Lys Tyr Ile Met Asp Gly
                85                  90                  95

Ile Lys Trp Ala Asn Asp Tyr Phe Ile Lys Cys Asn Pro Thr Pro Gly
            100                 105                 110

Val Tyr Tyr Tyr Gln Val Gly Asp Gly Gly Lys Asp His Ser Trp Trp
        115                 120                 125

Gly Pro Ala Glu Val Met Gln Met Glu Arg Pro Ser Phe Lys Val Asp
    130                 135                 140

Ala Ser Lys Pro Gly Ser Ala Val Cys Ala Ser Thr Ala Ala Ser Leu
145                 150                 155                 160

Ala Ser Ala Ala Val Val Phe Lys Ser Ser Asp Pro Thr Tyr Ala Glu

                165                 170                 175

Lys Cys Ile Ser His Ala Lys Asn Leu Phe Asp Met Ala Asp Lys Ala
            180                 185                 190

Lys Ser Asp Ala Gly Tyr Thr Ala Ala Ser Gly Tyr Tyr Ser Ser Ser
        195                 200                 205

Ser Phe Tyr Asp Asp Leu Ser Trp Ala Ala Val Trp Leu Tyr Leu Ala
    210                 215                 220

Thr Asn Asp Ser Thr Tyr Leu Asp Lys Ala Glu Ser Tyr Val Pro Asn
225                 230                 235                 240

Trp Gly Lys Glu Gln Gln Thr Asp Ile Ile Ala Tyr Lys Trp Gly Gln
                245                 250                 255

Cys Trp Asp Asp Val His Tyr Gly Ala Glu Leu Leu Leu Ala Lys Leu
            260                 265                 270

Thr Asn Lys Gln Leu Tyr Lys Asp Ser Ile Glu Met Asn Leu Asp Phe
        275                 280                 285

Trp Thr Thr Gly Val Asn Gly Thr Arg Val Ser Tyr Thr Pro Lys Gly
    290                 295                 300

Leu Ala Trp Leu Phe Gln Trp Gly Ser Leu Arg His Ala Thr Thr Gln
305                 310                 315                 320

Ala Phe Leu Ala Gly Val Tyr Ala Glu Trp Glu Gly Cys Thr Pro Ser
                325                 330                 335

Lys Val Ser Val Tyr Lys Asp Phe Leu Lys Ser Gln Ile Asp Tyr Ala
            340                 345                 350

Leu Gly Ser Thr Gly Arg Ser Phe Val Val Gly Tyr Gly Val Asn Pro
        355                 360                 365

Pro Gln His Pro His His Arg Thr Ala His Gly Ser Trp Thr Asp Gln
    370                 375                 380

Met Thr Ser Pro Thr Tyr His Arg His Thr Ile Tyr Gly Ala Leu Val
385                 390                 395                 400

Gly Gly Pro Asp Asn Ala Asp Gly Tyr Thr Asp Glu Ile Asn Asn Tyr
                405                 410                 415

Val Asn Asn Glu Ile Ala Cys Asp Tyr Asn Ala Gly Phe Thr Gly Ala
            420                 425                 430

Leu Ala Lys Met Tyr Lys His Ser Gly Gly Asp Pro Ile Pro Asn Phe
        435                 440                 445

Lys Ala Ile Glu Lys Ile Thr Asn Asp Glu Val Ile Ile Lys Ala Gly
    450                 455                 460

Leu Asn Ser Thr Gly Pro Asn Tyr Thr Glu Ile Lys Ala Val Val Tyr
465                 470                 475                 480

Asn Gln Thr Gly Trp Pro Ala Arg Val Thr Asp Lys Ile Ser Phe Lys
                485                 490                 495

Tyr Phe Met Asp Leu Ser Glu Ile Val Ala Ala Gly Ile Asp Pro Leu
            500                 505                 510

Ser Leu Val Thr Ser Ser Asn Tyr Ser Glu Gly Lys Asn Thr Lys Val
        515                 520                 525

Ser Gly Val Leu Pro Trp Asp Val Ser Asn Asn Val Tyr Tyr Val Asn
    530                 535                 540

Val Asp Leu Thr Gly Glu Asn Ile Tyr Pro Gly Gly Gln Ser Ala Cys
545                 550                 555                 560

Arg Arg Glu Val Gln Phe Arg Ile Ala Ala Pro Gln Gly Thr Thr Tyr
                565                 570                 575

Trp Asn Pro Lys Asn Asp Phe Ser Tyr Asp Gly Leu Pro Thr Thr Ser
            580                 585                 590

Thr Val Asn Thr Val Thr Asn Ile Pro Val Tyr Asp Asn Gly Val Lys
        595                 600                 605

Val Phe Gly Asn Glu Pro Ala Gly Gly Ser Glu Asn Pro Asp Pro Glu
    610                 615                 620

Ile Leu Tyr Gly Asp Val Asn Ser Asp Lys Asn Val Asp Ala Leu Asp
625                 630                 635                 640

Phe Ala Ala Leu Lys Lys Tyr Leu Leu Gly Gly Thr Ser Ser Ile Asp
                645                 650                 655

Val Lys Ala Ala Asp Thr Tyr Lys Asp Gly Asn Ile Asp Ala Ile Asp
            660                 665                 670

Met Ala Thr Leu Lys Lys Tyr Leu Leu Gly Thr Ile Thr Gln Leu Pro
        675                 680                 685

Gln Gly Leu Glu His His His His His His
    690                 695

<210>  17
<211>  1434
<212>  DNA
<213>  Escherichia coli

<400>  17
atgtatgatg cttcacttat tccgaatctt cagattccac aaaagaacat tccgaataat      60

gatggaatga attttgtaaa aggtttaaga ctcggatgga atctgggtaa tacatttgat     120

gcttttaacg gtacaaatat tactaatgaa ttggattatg aaacatcatg gagcggtatc     180

aaaacaacta agcagatgat agatgcaata aagcaaaaag gattcaatac tgttcgtatt     240

cctgtatcct ggcatccaca cgtaagtggt tcagattaca aaatcagtga tgtatggatg     300

aatcgtgttc aagaagtagt aaattattgt atagataata aaatgtatgt cattttaaac     360

acacatcatg acgttgacaa agtaaaaggt tatttcccaa gcagtcaata tatggcaagc     420

tccaagaaat atataactag tgtctgggca cagattgctg ctaggtttgc aaactatgat     480

gagcatctta tttttgaagg aatgaacgag cctcgtcttg taggacatgc aaatgagtgg     540

tggcctgagc tgacaaattc agatgtagtt gattctatta attgtattaa tcaacttaat     600

caggattttg ttaatacagt acgtgcaaca ggtggaaaaa atgcaagcag atatcttatg     660

tgtccaggat atgttgcatc tcctgacgga gcaacaaacg attacttcag aatgccaaat     720

gatatttctg gtaataacaa caaaataatt gtatctgtac atgcatattg tccatggaat     780

tttgcagggt tggcaatggc tgatggaggt acaaatgctt ggaatataaa tgattcaaaa     840

gatcaaagtg aagttacttg gtttatggat aatatttata ataagtatac aagcaggggt     900

attcctgtaa taatcggtga atgtggagca gtagataaga acaatctgaa gacaagagta     960

gaatatatgt cctattatgt tgcacaagct aaagcacgtg gtatattatg catattgtgg    1020

gataacaata atttctcagg tactggtgaa ttatttggtt tcttcgatag aagaagctgt    1080

cagttcaagt tccctgaaat tatagatgga atggtgaaat atgctttcga agccaagaca    1140

gatcctgacc ccggcacaaa gctcgttcct acatggggcg atacaaactg cgacggcgtt    1200

gtaaatgttg ctgacgtagt agttcttaac agattcctca acgatcctac atattctaac    1260

attactgatc agggtaaggt taacgcagac gttgttgatc ctcaggataa gtccggcgca    1320

gcagttgatc ctgcaggcgt aaagctcaca gtagctgact ctgaggcaat cctcaaggct    1380

atcgttgaac tcatcacact tcctcaactc gagcaccacc accaccacca ctga          1434

<210>  18
<211>  477
<212>  PRT
<213>  Escherichia coli

<400>  18

Met Tyr Asp Ala Ser Leu Ile Pro Asn Leu Gln Ile Pro Gln Lys Asn
1               5                   10                  15

Ile Pro Asn Asn Asp Gly Met Asn Phe Val Lys Gly Leu Arg Leu Gly
            20                  25                  30

Trp Asn Leu Gly Asn Thr Phe Asp Ala Phe Asn Gly Thr Asn Ile Thr
        35                  40                  45

Asn Glu Leu Asp Tyr Glu Thr Ser Trp Ser Gly Ile Lys Thr Thr Lys
    50                  55                  60

Gln Met Ile Asp Ala Ile Lys Gln Lys Gly Phe Asn Thr Val Arg Ile
65                  70                  75                  80

Pro Val Ser Trp His Pro His Val Ser Gly Ser Asp Tyr Lys Ile Ser
                85                  90                  95

Asp Val Trp Met Asn Arg Val Gln Glu Val Val Asn Tyr Cys Ile Asp
            100                 105                 110

Asn Lys Met Tyr Val Ile Leu Asn Thr His His Asp Val Asp Lys Val
        115                 120                 125

Lys Gly Tyr Phe Pro Ser Ser Gln Tyr Met Ala Ser Ser Lys Lys Tyr
    130                 135                 140

Ile Thr Ser Val Trp Ala Gln Ile Ala Ala Arg Phe Ala Asn Tyr Asp
145                 150                 155                 160

Glu His Leu Ile Phe Glu Gly Met Asn Glu Pro Arg Leu Val Gly His
                165                 170                 175

Ala Asn Glu Trp Trp Pro Glu Leu Thr Asn Ser Asp Val Val Asp Ser
            180                 185                 190

Ile Asn Cys Ile Asn Gln Leu Asn Gln Asp Phe Val Asn Thr Val Arg
        195                 200                 205

Ala Thr Gly Gly Lys Asn Ala Ser Arg Tyr Leu Met Cys Pro Gly Tyr
    210                 215                 220

Val Ala Ser Pro Asp Gly Ala Thr Asn Asp Tyr Phe Arg Met Pro Asn
225                 230                 235                 240

Asp Ile Ser Gly Asn Asn Asn Lys Ile Ile Val Ser Val His Ala Tyr
                245                 250                 255

Cys Pro Trp Asn Phe Ala Gly Leu Ala Met Ala Asp Gly Gly Thr Asn
            260                 265                 270

Ala Trp Asn Ile Asn Asp Ser Lys Asp Gln Ser Glu Val Thr Trp Phe
        275                 280                 285

Met Asp Asn Ile Tyr Asn Lys Tyr Thr Ser Arg Gly Ile Pro Val Ile
    290                 295                 300

Ile Gly Glu Cys Gly Ala Val Asp Lys Asn Asn Leu Lys Thr Arg Val
305                 310                 315                 320

Glu Tyr Met Ser Tyr Tyr Val Ala Gln Ala Lys Ala Arg Gly Ile Leu
                325                 330                 335

Cys Ile Leu Trp Asp Asn Asn Asn Phe Ser Gly Thr Gly Glu Leu Phe
            340                 345                 350

Gly Phe Phe Asp Arg Arg Ser Cys Gln Phe Lys Phe Pro Glu Ile Ile
        355                 360                 365

Asp Gly Met Val Lys Tyr Ala Phe Glu Ala Lys Thr Asp Pro Asp Pro
    370                 375                 380

Gly Thr Lys Leu Val Pro Thr Trp Gly Asp Thr Asn Cys Asp Gly Val
385                 390                 395                 400

Val Asn Val Ala Asp Val Val Val Leu Asn Arg Phe Leu Asn Asp Pro
                405                 410                 415

Thr Tyr Ser Asn Ile Thr Asp Gln Gly Lys Val Asn Ala Asp Val Val
            420                 425                 430

Asp Pro Gln Asp Lys Ser Gly Ala Ala Val Asp Pro Ala Gly Val Lys
        435                 440                 445

Leu Thr Val Ala Asp Ser Glu Ala Ile Leu Lys Ala Ile Val Glu Leu
    450                 455                 460

Ile Thr Leu Pro Gln Leu Glu His His His His His His
465                 470                 475